# EUROPEAN PATENT APPLICATION

(11) **EP 1 104 813 A1**
(43) Date of publication of application: **06.06.2001**
(21) Application number: 99204064.2
(22) Date of filing: 01.12.1999
(51) Int. Cl.: C12N 15/86

(54) **Conditional replication of recombinant human adenovirus DNA carrying modified inverted terminal repeat sequences**

(71) Applicant: Leids Universitair Medisch Centrum, 2333 ZA Leiden (NL)
(72) Inventor: Rademaker, Hendrik Jan, 2311 HM Leiden (NL); Fallaux, Frits Jacobus, 2353 KK Leiderdorp (NL); De Jong, Robert Niels, 3562 KG Utrecht (NL); Van der Vliet, Pieter Charles, 3914 ZS Doorn (NL); Hoeben, Robert Cornelis, 2314 EX Leiden (NL)
(74) Representative: Ottevangers, Sietse Ulbe

(57) **Abstract**

The present application relates to recombinant adenoviral vectors (rAds) that are not complemented in target cells. The rAds are carrying modified terminal repetitions (ITRs) to prevent replication initiation of those vectors that were trans-complemented by wild-type human adeno viruses (Ads). For that purpose the replication core origin of a CELO, an Ad that infects avian species, is combined with a human rAd. The resulting vectors are termed rescue-defective Ads (rdAds). The sequence of the replication core origin of the CELO Phelps strains (CELO^{PHELPS}) shows the presence of cytosines instead of guanins at positions one, four, and seven when compared to human Ad5.

## Description

The invention relates to the field of human and veterinary medicine, more particularly to adenoviral vectors to be used in gene therapy and vaccination, and methods for production thereof.

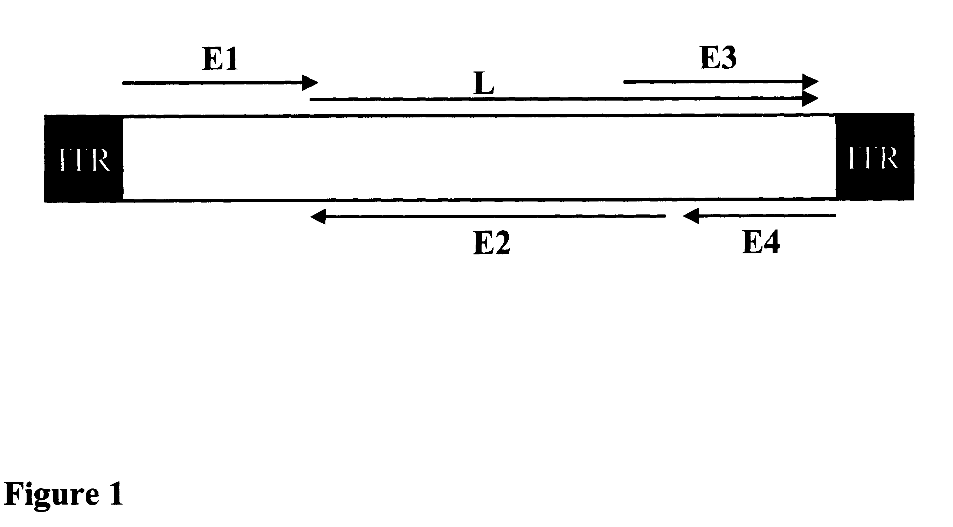

Human adenoviruses (Ads) are non-enveloped, icosahedral DNA viruses causing predominantly respiratory diseases, conjunctivitis, and intestinal infections in their natural host. The genome consists of a linear, double-stranded DNA molecule of about 36 kb carrying inverted terminal repetitions (ITR) (Horwitz, 1990). Infection of human cells by Ads results in a lytic and productive infection. During the lytic cycle, the entire genetic program of the virus is expressed, and this eventually leads to the production of progeny virus and the death of the host cell. During the productive infection cycle, the viral genes are expressed in two phases: the early phase, which is the period up to the onset of viral DNA replication, and the late phase, which starts with the initiation of viral DNA replication. During the early phase only the early gene products, encoded by early regions E1, E2, E3 and E4, are expressed, which carry out a number of functions that prepare the cell for synthesis of viral structural proteins (Berk, 1986). During the late phase the late viral gene products are expressed in addition to the early gene products and host-cell DNA and protein synthesis are shut off. Consequently, the cell becomes dedicated to the production of viral DNA and of viral structural proteins (Tooze, 1981). Reprogramming of the infected cell in the early phase starts with the expression of early la (E1A) gene (Nevins *et al.,* 1979; Rowe *et al.,* 1984). The main functions of the E1A gene products are (i) to induce quiescent cells to enter the cell cycle and resume cellular DNA synthesis, and (ii) to transcriptionally activate the E1B gene and the other early regions (E2, E3, E4). A schematic representation of the adenovirus genome is depicted in figure 1.

Although Ads can replicate only in cells of humans, chimpanzees, and cotton rats, they can infect cells from many other species as well. Infection of rodent cells, that are non- or semi-permissive to Ad replication, results in a mainly abortive infection. Although little or no progeny virus is produced during an abortive infection, at least part of the viral genome is expressed. A small fraction of the abortively infected cells may incorporate Ad DNA in their genome, which leads to the generation of transformed cells. This transformation process essentially depends on the functions encoded by early region E1, consisting of the E1A and E1B genes. Cells that are transformed by Ad constitutively express E1A and E1B proteins, proliferate in an uncontrolled manner and, in most cases, have an infinite life span. Furthermore, Ad-transformed cells are usually able to induce tumors when transplanted into certain hosts.

Adenoviruses have been classified according to their potential to induce tumors in hamsters into class A viruses (e.g. Adl2) which are highly oncogenic, class B viruses (e.g. Ad7) which are weakly oncogenic, and class C viruses (e.g. Ad2 and Ad5) which are non-oncogenic. The serotypes that have been used for vector development (Ad2 and Ad5) are not associated with severe human pathology (Horwitz, 1990). Gene-transfer vectors derived from Ads (so-called recombinant adenoviral vectors (rAds) have a number of features that make them particularly useful for gene-transfer purposes: the biology of Ads has been characterized in detail, the virus is extremely efficient in introducing its DNA into the host cell, Ads can infect a wide variety of dividing and non-dividing cells of a broad range of species, and the virus can be produced in large quantities with relative ease. In contrast to retroviruses, Ads do not integrate into the host cell genome.

Recombinant adenoviruses are among the most efficient vectors for gene therapy purposes, and have rapidly become the vector of choice in many human gene therapy protocols. The rAd is used as a vehicle for transport of a therapeutic gene. For most applications, replication of the vector is undesirable (e.g. for the treatment of genetic disorders). Therefore, the majority of rAds currently used in gene therapy have a deletion in early region 1 (E1) of the viral genome, where novel genetic information can be introduced. The E1 deletion renders the recombinant virus replication-defective, and thus provides an important safety feature. E1-deleted rAd can be propagated on dedicated helper cells, specialized cells that provide the E1 functions in *trans,* such as cell lines 293 (Graham *et*. *al.,* 1977), 911 (Fallaux *et. al.,* 1996), and PER.C6 (Fallaux et. *al.,* 1998). Although encouraging results have been obtained sofar with rAd, two major problems are associated with the use of rAds: viz. the host immune response against the adenovirus particles and the transduced cells, and the generation of replication-competent adenovirus (RCA) during manufacture of rAd lots. RCA are revertant vectors that reacquired the E1 region as a result of homologous recombination with E1 sequences integrated in the helper cells.

Some rAds are also deprived of E3 sequences in order to increase the amount of heterologous sequences that can be accommodated in the vector. None of the Ad E3 gene-products are required for replication in cultured cells. The E3-encoded proteins do, however, play an important role in Ad multiplication *in vivo,* since they protect infected cells from being eradicated by the host's immune-response. The E3 19-kDa protein, which is synthesized abundantly in infected cells, associates intracellularly with the heavy chain of the class I major histocompatibility complex (MHC), thereby abrogating the intracellular transport and post-translational modification of the class I antigens (Signas *et al.,* 1982; Burgert and Kvist, 1985; Andersson *et al.,* 1985). As a result, cell-surface expression of class I antigens is decreased, rendering the infected cells less sensitive to the class I MHC-restricted immune-response. The E3 14-kDa proteins have been shown to render the otherwise very susceptible virus-infected cells resistant to cytolysis by tumour necrosis factor (Gooding and Wold, 1990).

Currently, cell line 293 has been used world wide for the propagation of E1-deleted rAds. The 293 cell line was generated by transformation of HEK cells with fragmented Ad5 DNA. These cells contain and express the left 11% of the Ad5 genome (Graham *et al.,* 1977; Graham and Prevec, 1991). The 293 cell line can be used to produce high-titer rAd [up to 10¹²-10¹³ plaque-forming units (PFU)/ml], and can be used to determine the titer of E1-deleted rAd stocks in a plaque assay. Recently, we reported on the generation and characterization of an alternative helper cell line, human cell line 911 (Fallaux *et al.,* 1996). The 911 cell line was generated by transformation of HER cells with a plasmid containing basepairs (bp) 79 - 5789 of the Ad5 genome. The advantages of the 911 cell line are its defined Ad5-DNA content, and the outstanding performance in plaque assays.

As stated above, the 911 cell line has several characteristics that make it superior to the commonly used 293 cells. However, an important drawback of the 911 cell line is, like has been shown for 293 (Lochmuller *et al.,* 1994, Hehir *et al.,* 1996), the potential generation of replication-competent adenoviruses (RCA). The presence of RCA in rAd-batches is to be avoided because RCA (i) will replicate in an uncontrolled fashion; (ii) can complement rAd, causing uncontrolled multiplication of the vector and (iii) rAd-batches containing RCA induce significant tissue damage and hence strong pathological side effects (Lochmuller *et al.,* 1994). Consequently, all rAd-batches are routinely checked for the presence of RCA.

The major cause of RCA production is homologous recombination between overlapping sequences from the recombinant vector and the adenovirus constructs in the helper cells (Hehir *et al.,* 1996). Therefore, it was expected that generation of RCA should be prevented by elimination of sequence homology between the vector DNA and the Ad sequences in the genome of the helper cells. This has been realized by the construction of new adenovirus vectors from which all E1 encoding sequences are deleted, and by the development of helper cells (PER.C6) that contain E1-encoding sequences only (Fallaux *et al.,* 1998). PER cells were generated by transformation of HER cells with a plasmid that contain Ad5 bp 459-3510. All vectors that have a deletion spanning at least Ad bp 459 to 3510 have no sequence homology with PER cells. So far, with this vector/cell-line combination, generation of RCA by homologous recombination has not been observed.

Another problem that is associated with the use of the first-generation rAds is the host-defense reactions against treatment with adenovirus. This is caused by the strong immunogenicity of the virus particle, and by the illicit expression of adenovirus genes that reside in the El-deleted vectors, resulting in a Cytotoxic T-cell (CTL) response against the transduced cells. *In-vivo* administration of Ad particles triggers the development of neutralizing antibodies by the host. As a result, a subsequent administration of the virus will be less effective or even completely ineffective. Experiments performed by Yang and collaborators have demonstrated that after adenovirus-mediated gene transfer into immunocompetent animals, the expression of the transgene gradually decreases and disappears approximately 2- 4 weeks post-infection (Yang *et al.,* 1994a; Yang *et al.,* 1994b). They demonstrated that this was caused by the development of a CTL response against the transduced cells. The CTLs were directed against adenovirus proteins expressed by the viral vectors. The transduced cells synthesized the 72-kDa DNA-binding protein (DBP, the product of the E2A gene), as well as the late-gene products penton and fiber. These Ad proteins, encoded by the rAd, were expressed despite deletion of the E1 region. This demonstrates that deletion of the E1 region is not sufficient to completely inhibit expression of the other viral genes (Engelhardt *et al.,* 1994a). The CTL response eradicates the transduced cells and causes inflammatory reactions (Bout *et al.,* 1994a; Engelhardt *et al.,* 1993; Simon *et al.,* 1993). As this adverse reaction is hampering gene therapy, several solutions to this problem have been suggested, such as (i) the use of immunosuppressive agents after treatment with the rAd, (ii) retainment of the Ad E3 region in the vector, and (iii) the use of temperature-sensitive (ts) mutants of Ad, which have a point mutation in the E2A region. The E2A protein plays a pivotal role in the switch to the synthesis of late adenovirus proteins. Therefore, it is attractive to make recombinant adenoviruses that are mutated in the E2 region, rendering it temperature sensitive. A well-known example of a ts Ad mutant is ts125. The ts125 viruses have a point mutation in the E2A gene (Ensinger and Ginsberg, 1972; Kruijer *et al.,* 1981, 1983). The mutant DBP has a wild-type conformation and activity at 32 °C, but is unstable at 38-40 °C. ts125 mutant recombinant adenovirus have been tested, and prolonged recombinant gene expression was reported (Yang *et al.,* 1994b; Engelhardt et *al.,* 1994a; Engelhardt *et al.,* 1994b; Yang *et al.,* 1995). However, pathology in the lungs of cotton rats was still high (Engelhardt *et al.,* 1994a), indicating that the use of ts mutants yields only a partial improvement for recombinant adenovirus technology. This may be caused by the fact that the lung temperature (34-35 °C) may not be restrictive for ts125-DBP activity. In addition, the immunogenic protein is being synthesized, even though it is unstable at the non-permissive temperature. Furthermore, it is to be expected that the unstable protein may still activate late gene expression to some extent. An additional difficulty associated with the use of ts125 mutant adenoviruses is that a high frequency of reversion is observed. These revertants are either real revertants or second-site mutations (Kruijer *et al.,* 1983; Nicolas *et al.,* 1981). Both types of revertants yield an E2A protein that functions at normal temperature and have therefore similar toxicity as the wild-type virus. A solution to this problem may be to delete the E2A coding sequences from the rAd genome but express them in the helper cell lines. Alternatively, vectors lacking E2B as well as E1 and E3, so that the Ad DNA polymerase and terminal protein (both essential for replication of the viral genome) are not present, appear to be less immunogenic and have been shown to improve transgene persistence (Amalfitano *et al.,* 1998).

The construction of 293 derivatives that express E4 as well as E1 has allowed the removal of the E4 region from the rAd, which effectively blocks DNA replication and late gene expression in the target cells (refs Wang *et al.,* 1995; Krougliak and Graham, 1995; Yeh *et al.,* 1996). Deletion of the E4 region further increases the size of foreign DNA that can be cloned in the vector. However, there is no consensus as yet on the effect of E4 deletion on prolonging transgene expression (reviewed by Leppard, 1997).

The maximum amount of heterologous sequences that can be accommodated in the current rAds is approximately 8 kilobases (kb). Bett and collaborators (1993) have demonstrated that adenoviruses can encapsidate DNA of up to 105% of the normal genome size. Larger genomes tend to be unstable resulting in loss of DNA sequences during propagation of the virus. Combining deletions in the E1 and E3 regions of the viral genomes allows the introduction of foreign DNA to a maximum of approximately 8.3 kb. The insertion capacity can be further increased by deleting the E4 region (Krougliak and Graham, 1995; Yeh *et al.,* 1996). However, increasing the insertion capacity of the rAd is a difficult task, since it requires the introduction and expression of the deleted Ad sequences in the helper cells. Major hurdles in this approach are (i) that the Ad genes should be expressed to very high levels, and (ii) that the presence of the Ad gene products is potentially toxic for the cells. The potential toxicity can, however, be avoided by the use of inducible promoters that are switched on only after infection with the vector (Krougliak and Graham, 1995; Wang *et al.,* 1995). Alternatively, one can use a helper virus. This approach allows the construction and application of so-called "gutless" adenovirus vectors (gAds) that contain only the Ad encapsidation signal and the ITRs, creating an insertion capacity up to 38 kb (e.g. Mitani *et al.,* 1995). Gutless adenovirus vectors are also referred to as minimal or helper-dependent adenovirus vectors. gAd DNA can be transferred into the helper cell line by transfection, which is subsequently infected with the helper virus. The helper virus provides the Ad proteins that are required for replication and packaging of the gAd vector. A drawback of this strategy is that the helper cells will produce gAds particles as well as helper viruses. The minimal vector particles can be separated from the helper viruses by density-gradient centrifugation, provided that the size of the minimal vector genome is well below that of the helper virus. Recently, however, an alternative strategy, employing the bacteriophage P1 Cre/loxP recombination system, has been developed (reviewed by Morsy and Caskey, 1999) that allows the production of gAds carrying wild-type-sized DNA. The future application of gAds is expected to reduce the immunological responses by the host, and prolong the expression of the transferred gene.

### Adenovirus replication

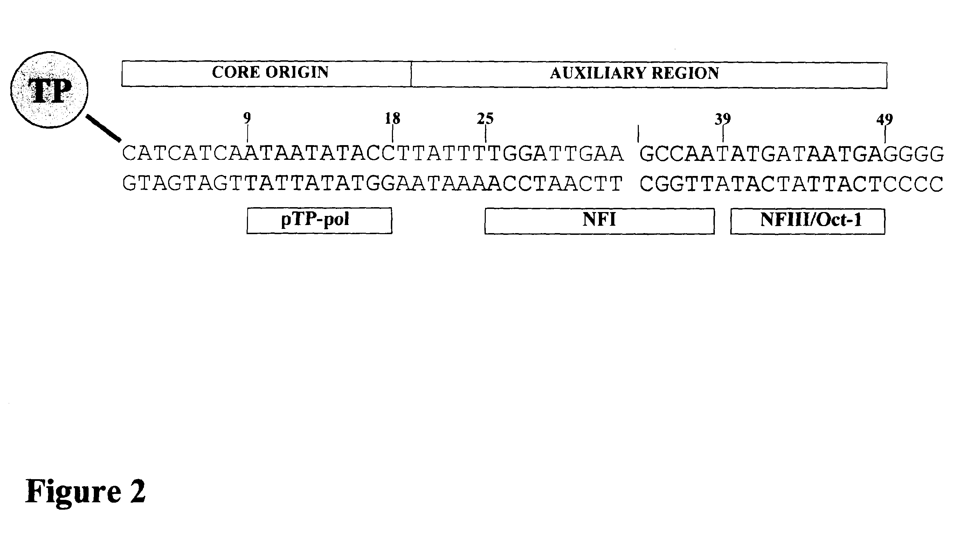

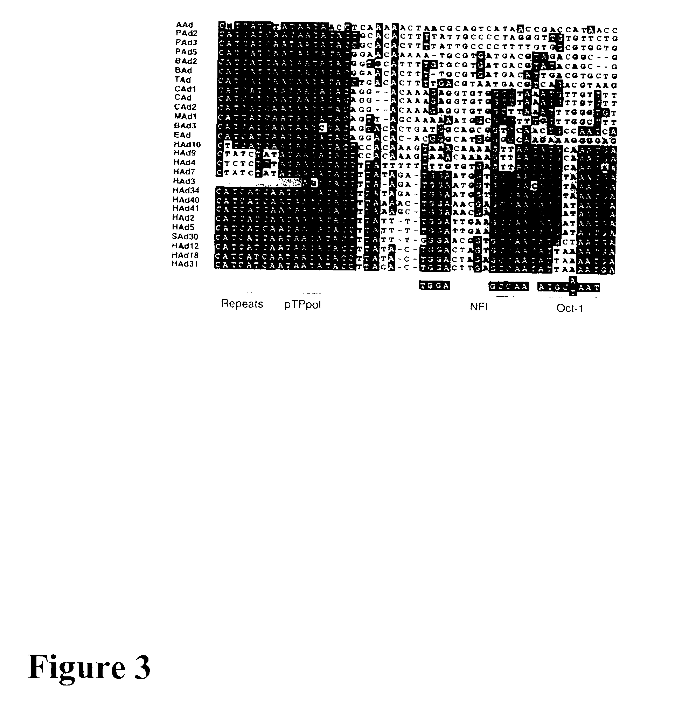

The human adenovirus (Ad) genome is a linear double-stranded DNA molecule of approximately 36,000 base pairs with the 55-kDa terminal protein (TP) covalently bound to the 5'-terminus of each strand. The Ad DNA contains identical Inverted Terminal Repeats (ITR) of about 100 to 160 base pairs with the exact length depending on the serotype (Van Ormondt and Galibert, 1984). The viral origins of replication are located within the ITRs at the genome ends. The ITR sequence of human adenovirus serotypes 2 (Ad2) and 5 (Ad5) shows the presence of a region that is conserved in all serotypes between nucleotide (nt) 9 and 18 that constitutes the core origin (Fig.2). Two other regions in the ITR are mainly conserved in the human Ad serotypes and are located in the auxiliary region of the origin which enhance replication up to 200-fold both *in vitro* and *in vivo* (Rosenfeld *et al.,* 1987; Wides *et al.,* 1987; Challberg and Rawlins, 1984; Hay, 1985; Lally *et al.,* 1984). These regions bind the cellular transcription factors nuclear factor 1 (NFI) and NFIII/Oct-1 (Fig. 2; Nagata *et al.,* 1983; Pruijn *et al.,* 1986). Comparison of Ad origins isolated thus far from a variety of species shows that Oct-1 and NFI binding sites are lacking in non-human and primate Ads (Fig.3). The cellular factors which bind to the origin may determine the host range of the various Ad serotypes.

Ad DNA replication involves the formation of a large nucleoprotein complex at the origin, that is stabilized by multiple DNA-protein and protein-protein interactions. Replication of the viral DNA starts at both ends of the virus genome employing a protein-priming mechanism. The Ad precursor terminal protein (pTP) acting as the protein primer becomes covalently linked to the first nucleotide, usually a dCTP residue, *via* a phosphodiester bond to the β-OH group of a serine residue. The 3'-OH group of the pTP-dCMP complex is the primer for the Ad polymerase (pol) to begin strand synthesis. Replication of Ad5 initiates at the fourth base (C4), rather than at position 1 (C1) at the very end of the linear DNA (King and van der Vliet, 1994). Initiation at C4 is followed by the formation of a trinucleotide intermediate, pTP-CAT, guided by the template G4T5A6 sequence. This is followed by a "jump" of this product to position 1-3 at the end of the DNA, only then followed by elongation. Sequence analyses of other Ad serotypes suggests that jumping two or four bases also occurs. DNA chain elongation proceeds *via* a strand displacement mechanism and requires the Ad DNA binding protein (DBP) .

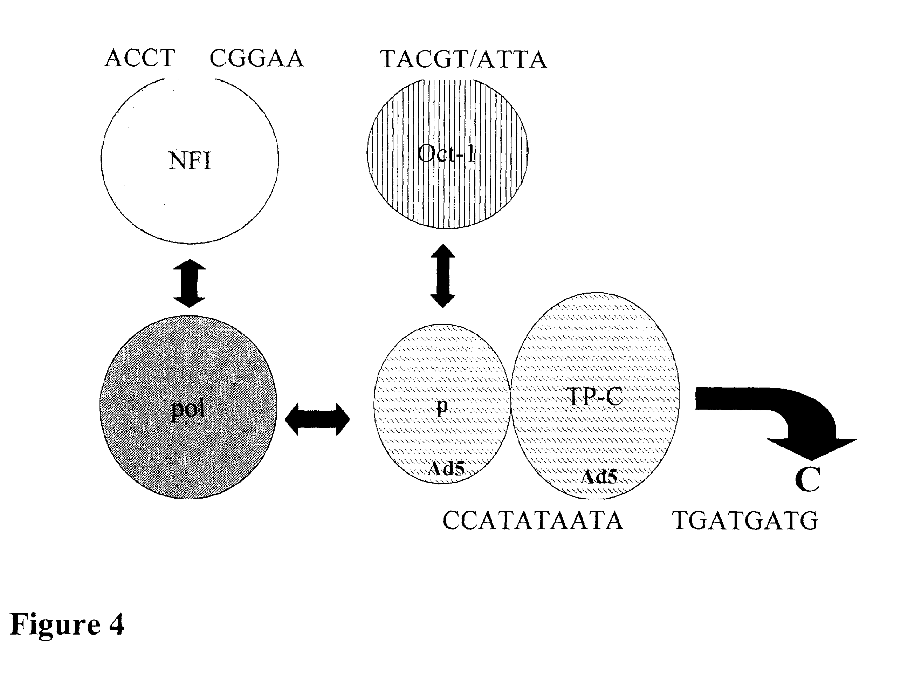

Adenovirus DNA replication has been studied extensively, and was one of the first replication systems that could be reconstituted *in vitro* (reviewed by Van der Vliet, 1995). Initiation of Ad5 DNA replication requires two virus-encoded proteins, the 80-kDa pTP and the 140-kDa pol. In addition, two cellular transcription factors, NFI and NFIII/Oct-1, which bind specifically to the auxiliary region next to the core origin (Fig.3), have been shown to stimulate the initiation reaction. The pTP-pol complex bound to the origin is secured on two sites: the polymerase interacts with NFI and pTP interacts with Oct-1. For all protein-protein interactions to occur, the Ad origin must be bent considerably (Fig.4).

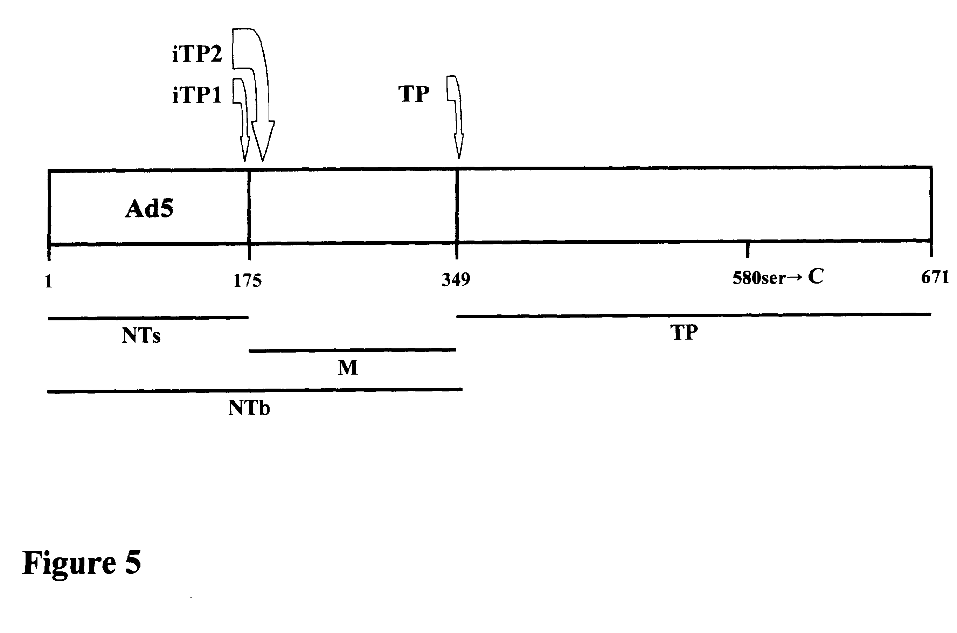

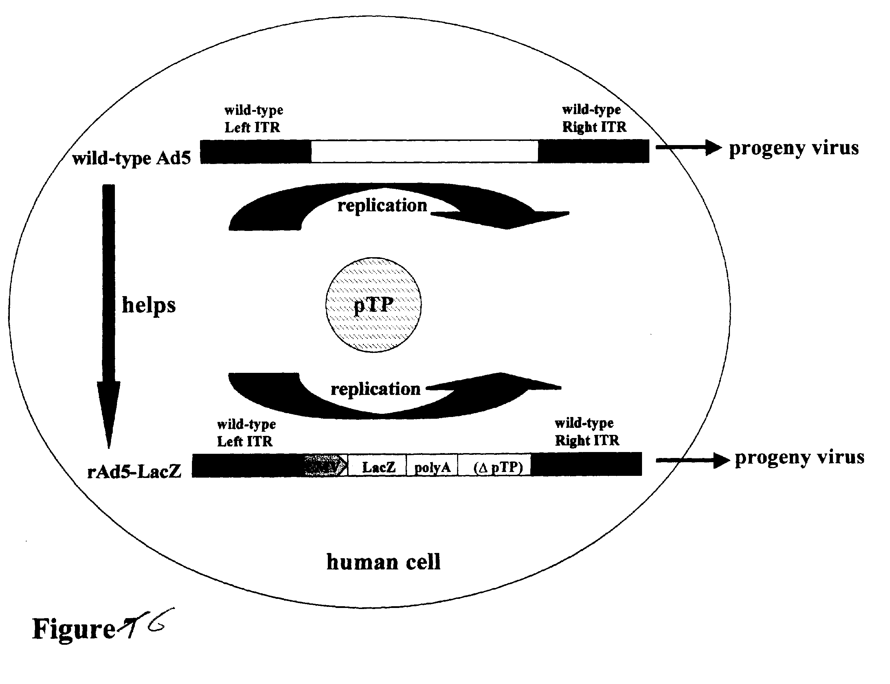

The adenovirus TPs are highly conserved in the various serotypes. The pTP becomes attached to the DNA as a consequence of protein priming and is proteolytically cleaved (Fig. 5) by the viral protease, leading to a shut-down of replication late in infection. It has been difficult to identify specific regions required for the various functions of pTP. Single mutations scattered over the entire protein seem to eliminate most functions simultaneously, possibly by changing the overall structure of pTP (Freimuth and Ginsberg, 1986; Fredman *et al.,* 1991; Roovers *et al.,* 1991). However, it has found that the amino-terminal precursor region of pTP is essential both for priming activity, DNA binding, and for interaction with the polymerase (Pettit *et al.,* 1989; Hay, 1995; Webster *et al.,* 1997). Recently, it has been shown that this region is also responsible for the interaction with Oct-1 (Botting and Hay, 1999). However, neither the interaction domain of pTP and Oct-1, nor that of pTP and pol has been mapped at the amino-acid level. The C-terminus of pTP is involved in attachment to the nuclear matrix (Fredman and Engler, 1993) and contains the essential amino acid serine (ser) 580 (Ad5) which binds the first dCMP residue during replication initiation.

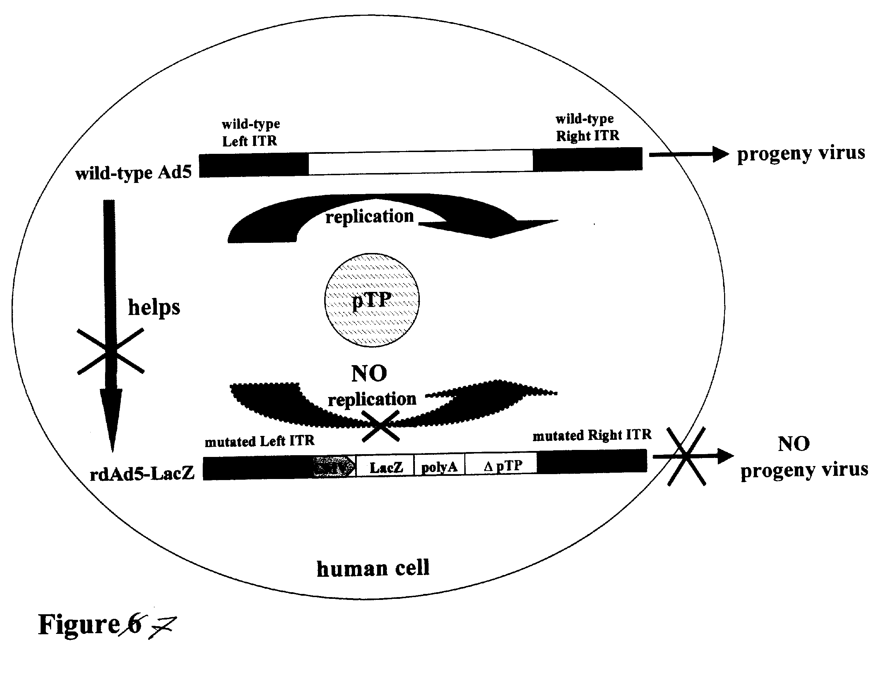

The majority of current Ad vectors used in gene therapy have a deletion in El of the viral genome, where novel genetic information can be introduced. These vectors can, like the wild-type Ad, infect various human cell-types, but cannot replicate autonomously, due to the E1 deletion. This provides an important safety feature. Superinfection by wild-type Ads could provide the missing E1 functions *in trans,* thus allowing the recombinant Ad vector to replicate (Fig. 6). This "rescue" or complementation of the defective recombinant vector by wild-type Ads has raised concerns about the possible dissemination and spreading of the vector (Imler *et al.,* 1995). Rescue of the vector may increase the amount of vector shed by the patient. In addition, uncontrolled replication and/or spreading of the vector in the patient could induce toxicity as a result of transgene expression in non-target tissues. Also, recombination between the recombinant vector and the wild-type Ad could occur, leading to viruses with unwanted properties. A particularly worrying recombination event would generate a replication-competent Ad carrying both E1 and the transgene. Furthermore, Ads are known to interact with several other DNA viruses (Horwitz, 1990)), and infection by pappilloma virus or cytomegalovirus could also result in the rescue of an E1-deleted Ad vector (Tevethia *et al.,* 1987; Phelps *et al.,* 1988).

A non-limiting example where the matter of rescue of the Ad vector immediately springs to the attention is gene therapy for the treatment of patients with cystic fibrosis (CF): the Ad-CFTR vector is administered to the airways, which is the natural target tissue of wild-type Ads. CF-patients treated with Ad-CFTR could be exposed to wild-type Ad infection in the period following the treatment. Therefore, CF-patients enrolled in clinical trials are screened for the presence of Ads by culture of the pharyngeal or nasal swabs on indicator cells, and by PCR for Ad E1 sequences (Zabner *et al*., 1993; Crystal *et al.,* 1994).

Imler and co-workers have evaluated the risk of Ad-vector rescue by wild-type Ads (Imler *et al.,* 1995). They selected the cotton rat *(Sigmodon hispidus)* as a model since it is permissive for wild-type human Ad5 replication (Pacini *et al.,* 1984), and it has been shown to mimic the histopathology of Ad5 infection in humans (Prince et al., 1993). Complementation of an E1-deleted recombinant Ad vector (Ad-CFTR) was detected both *in vitro* and *in vivo.* In their *in vivo* model, wild-type Ad5 was administered seven days after delivery of Ad-CFTR. This mimics the situation of a CF-patient treated with Ad-CFTR and subsequently exposed to a wild-type Ad infection. It was clearly shown that the recombinant Ad can be rescued from the target cells by the wild-type virus, since ten days after virus administration, the presence of Ad-CFTR was detected in the pharyngeal fluids of the animals which were super-infected with Ad5, but not in those of the animals that only received the Ad-CFTR. To prevent trans-complementation of recombinant Ad vectors, Imler and co-workers (Imler *et al.,* 1995) studied whether mutation of the cis-acting regulatory sequences which control the encapsidation of the viral genome could further attenuate Ad-CFTR. They demonstrated that when cells are co-infected with wild-type virus and the mutated Ad-CFTR, the viral DNA containing the natural encapsidation signal is preferentially packaged, leading to a rapid dilution of the recombinant virus. The authors concluded that mutation of the packaging sequences of recombinant Ad viruses is a way to confer a selective disadvantage to those viruses when wild-type viruses are present.

In spite of efforts to prevent replication of adenovirus vectors in, and spread of adenovirus vectors from target cells, there is as yet no satisfactory solution to this problem. The problem lies in the fact that adenovirus vectors comprise deficiencies that can be complemented. El-deleted vectors may be complemented by E1-like proteins encoded by the cell and/or other viruses. In addition, upon infection of a target cell comprising an adenovirus vector, with a wild-type adenovirus, proteins encoded by said wild-type adenovirus can complement at least in part deficiencies in the current generation of adenovirus vectors, resulting in replication of the adenovirus vector and/or in spread of the adenovirus vector to other cells of the body and/or other individuals.

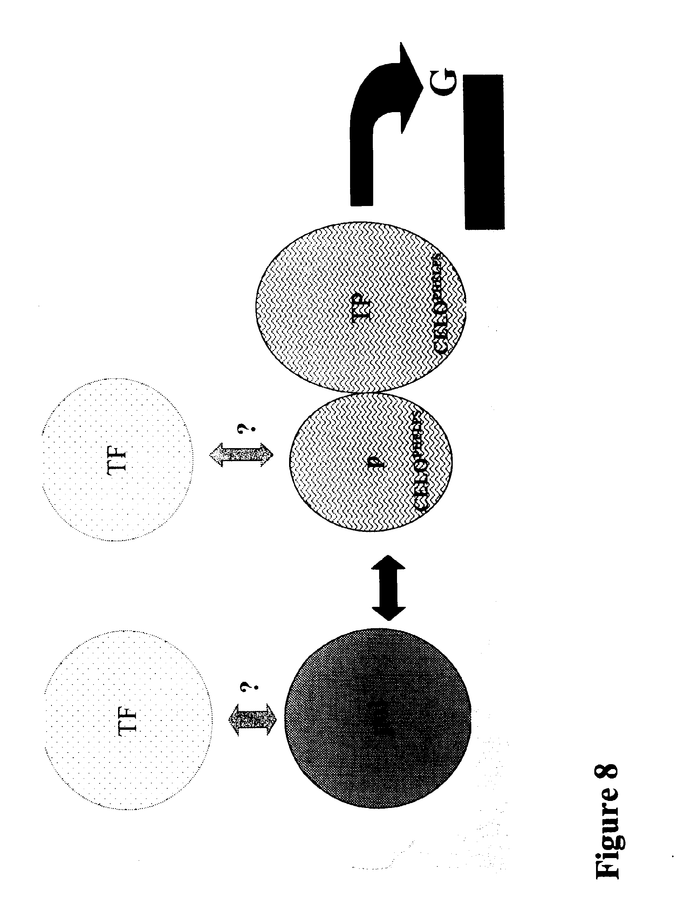

The invention now provides a solution to the problem of complementation of adenovirus vectors in target cells. The invention further provides means and methods to eliminate at least in part the rescue of recombinant Ads by wild-type adenoviruses. The system relies on the use of rAds carrying modified ITRs. These recombinant Ads are for the present invention termed rescue-defective Ads (rdAds). A detailed description of the experiments that were performed to develop the rdAd technology is given below. Briefly, rdAds can not be *trans*-complemented by most wild-type Ads due to mutation of the ITR sequences, which essentially prevents replication initiation by the replication machinery of at least some wild-type Ads commonly infecting the host species for which the rAds are intended to be used (Fig. 7).

Replication of the linear DNA genome of adenoviruses is achieved through the so-called protein priming replication initiation system. Replication of human Ads, including serotypes 2 and 5, which are commonly used for vector construction, starts with the binding of a dCMP residue to a serine in pTP. In fact, the majority of the known Ads, including animal Ads, initiate replication with the incorporation of a dCTP nucleotide. This is due to the fact that Ad ITRs are highly conserved (Fig. 3; Van Ormondt and Galibert, 1984). The Ad5 origin of replication sequence starts with 3'-G¹TAG⁴TAG⁷T-//. It has been shown previously that a pTP-CAT replication intermediate is formed using the template residues 4-6 (King and van der Vliet, 1994). This intermediate jumps back to position 1 of the template in order to proceed to elongation. The template sequence 3'-G¹TAG⁴TAG⁷T-// contains at least three positions (G1, G4, and G7) which can be used to start formation of a pTP-dCMP complex, and at least two positions (G1 and G4) to start pTP-CAT. Mutation of G1, G4, and G7 individually to C does not interfere with the formation of pTP-dCMP. However, mutation of all three G residues essentially completely abolishes the formation of pTP-dCMP, and thus replication (King and van der Vliet, 1994). Harris and Hay (1988) studied initiation of human adenovirus serotype 4 (Ad4) DNA replication on oligomer templates containing the terminal 18 nucleotides of the Ad4 minimal ori (shown in Fig. 3). They demonstrated that two mutant oligomers containing substitutions at positions 1, 4, and 7, or 4 and 7, within the repeats of the Ad4 minimal core origin did not support pTP-dCMP formation. Together, these data emphasize the crucial role of the sequence of the repeats within the adenovirus minimal origin of replication.

The large adenovirus family is divided by host range into Ads that infect mammals (the *Mastadenoviridae),* and Ads that infect avian species (the *Aviadenoviridae).* Chicken embryo lethal orphan (CELO) is classified as a fowl Ad type 1 (FAV-1), and can be isolated from healthy chickens. CELO has a genome of 43.8 kb, nearly 8 kb longer than the 35.9-kb genome of human Ads type 2 and 5 (Chiocca *et al.,* 1996). The CELO virus DNA has covalently attached terminal proteins, and has ITRs that are somewhat shorter than those of the *Mastadenoviridae* (Chiocca *et al.,* 1996). Interestingly, the sequence of the replication core origin repeats of the genome of the CELO Phelps strain (CELO^{PHELPS}), 3'-C¹TAC⁴TAC⁷T-, shows the presence of cytosines instead of guanines at positions one, four, and seven when compared to Ad5. Apparently, the CELO^{PHELPS} pTP can bind to dGMP (Fig. 8). In this respect, CELO ^{PHELPS} is unique: another CELO strain, ote, must start its replication with the binding of a dCMP residue, just like all other currently known Ads. The repeat-structure of the CELO^{PHELPS} origin (CTACTACT) strongly suggests that CELO^{PHELPS} also uses the jumping-back mechanism for its replication.

The CELO^{PHELPS} origin repeats (CTACTACT) can essentially not be used by pTP derived from for instance human adenovirus serotypes as a template to start replication. In the present invention this feature is used to generate adenovirus vectors that are complementation, i.e. rescue, defective in at least most animals species. The CELO^{PHELPS} origin is only a non-limiting example of an origin that may be used to generate a rescue defective adenovirus. (Part of) the (presumed) origin of replication sequence of various species are listed in table 1. Among these are several candidates (e.g. bacteriophage PRD1, mycoplasma virus P1) that can be used for the generation of the replication defective adenoviruses of the invention.

The origin of seven nucleotides may be extended with additional nucleotides as long as the addition of the nucleotides do not interfere with the initiation of replication characteristics of the origin in kind, not necessarily in amount. For instance the origin may be extended with additional three nucleotide repeats. A non-limiting example is one form of an extended CELO^{PHELPS} origin (CTACTACTACT). For the present invention an extended origin is considered a normal origin.

The 2,3, 5,6 positions in the origin are in several adenovirus serotypes, occupied by A and T nucleotides. These may be interchanged with other nucleotides as long as the substitutions do not interfere with the initiation of replication characteristics of the origin in kind, not necessarily in amount.

In one aspect the invention therefore provides a chimaeric adenoviral vector comprising a functional packaging signal from an adenovirus and comprising a site for protein primed replication initiation not present in the wild type adenovirus from which said packaging signal originates. A DNA strand in an adenovirus is usually double stranded. The site for protein primed replication initiation is therefore also usually a double stranded DNA. However, for the present invention when referring to a sequence in said site we refer to the sequence in the bottom strand calculated when the upper strand is depicted in the 5' to 3' orientation.

In a preferred embodiment said site for protein primed replication initiation is derived from a phage and/or a virus comprising an essentially linear DNA genome. Preferably, said virus is an adenovirus. In one embodiment said site for protein primed replication initiation preferably comprises a cytosine residue at at least two of the positions 1, 4, 7 and onward at intervals of three nucleotides. Preferably, said site for protein primed replication initiation comprises a cytosine residue at at least two of the positions 1, 4 and 7. Origins comprising cytosine residue at at least two of the positions 1, 4 and 7 and possibly onward, are typically used for the generation of vectors that are rescue defective in other species than the normal host of CELO^{PHELPS}, chickens. A preferred species is the human. Other preferred species are farm animals and animals that are held by humans for pleasure. Chickens are for origins based on CELO^{PHELPS} typically not preferred, although the chimaeric adenovirus of the invention based on CELO^{PHELPS} can be made essentially rescue defective in chickens. For instance through essentially eliminating the possibility of infection of the chickens by CELO^{PHELPS}, for instance through immunizing the chickens against CELO^{PHELPS}. An immunization strategy for CELO^{PHELPS} is possible since it is as yet the only recognized adenovirus capable of complementing an origin comprising cytosine residues. Typically, the possibility of essentially eliminating the possibility of infection is feasible when the number of wild type adenovirus serotypes capable of replicating and rescuing a chimaeric adenovirus vector of the invention and capable of infecting the host species for which the vector is intended to be used is limited. When the number of possible infecting virus strains is relatively large, such as for the human situation, it is preferred that the combination of the packaging signal and a site for protein primed replication initiation cannot be recognized by any single possible infecting virus strain, thereby limiting the chances of rescue of the chimaeric adenovirus, either due to the fact the packaging signal cannot be recognized and/or the site for protein primed replication initiation cannot be recognized. Preferably, the chimaeric vector of the invention comprises a site for protein primed replication initiation that cannot be recognized by at least the majority of the wild type adenoviruses that normally target the host for which the chimaeric virus is intended to be used, preferably a human host. Most preferably, the chimaeric vector of the invention comprises a site for protein primed replication initiation that cannot be recognized by any of the wild type adenoviruses that normally target the host for which the chimaeric virus is intended to be used.

In another embodiment the invention provides a chimaeric adenoviral vector of the invention wherein the adenovirus from which said packaging signal is derived comprises a site for protein primed replication initiation comprises a guanine residue at at least two of the positions 1, 4, 7 and onward at intervals of three nucleotides. Preferably said site for protein primed replication initiation comprises a guanine residue at at least two of the positions 1, 4 and 7. Particularly, for the human situation it is preferred that the adenovirus form which the packaging signal originates comprises guanine residue at the indicated positions at the site of protein primed replication initiation. Since most of the popular adenovirus vectors for delivery of genetic information in humans have such replication initiation sites the systems for producing such vectors in clinical grade quality can easily be adapted to accommodate also the production of the chimaeric vectors of the invention, without altering to a substantial amount the pharmacological properties of the vector once inside the patient. For instance all of the capsid proteins of the resulting chimaeric vector will be essentially the same as the rescue competent vector.

A chimaeric adenovirus vector of the invention typically but not necessarily further comprises at least a modification essentially preventing expression of El-region encoded proteins. Typically but not necessarily, such a modification comprises a deletion of at least part of the El-coding region. A chimaeric adenovirus vector of the invention typically but not necessarily further comprises a modification essentially preventing expression of the E2-region encoded protein pTP. A chimaeric adenovirus vector of the invention may comprise other modifications essentially preventing expression of other adenovirus encoded proteins.

For some purposes for instance where replication of the rescue defective adenovirus vectors of the invention, i.e. the chimaeric adenovirus of the invention, is desired, a precursor Terminal protein capable of binding to a site for protein primed replication initiation on said chimaeric vector and an adenovirus polymerase capable of binding to said precursor terminal protein is provided. Replication is for instance desired for the production of the rescue defective adenovirus vectors of the invention.

When the site for protein primed replication initiation comprises a sequence 3'-C¹TAC⁴TAC⁷T-// or a functional equivalent thereof, one possible precursor Terminal protein capable of binding to said site is a precursor Terminal protein comprising at least a nucleotide binding domain of CELO^{PHELPS} pTP.

The coding sequence of the CELO^{PHELPS} pTP gene was determined as following. Human adenovirus type 5 pTP was first identified as a 1959 bp open reading frame (nt 8583-10544, genbank ID m73260). When this open reading frame was cloned it encoded a non-functional pTP. Later it was identified that the full-length pTP was spliced and that the functional start codon was at nucleotide position 14119. By DNase protection assays, the splice sites that were used to produce functional pTP was determined between nt 14110 and nt 14111 for the splice donor site and between nt 10589 and nt 10590 for the splice acceptor site. After processing, the pTP mRNA is 2013 bp in length and encodes for 671 amino acids. When aligning the full length human Adenovirus type 5 pTP against pTP amino acid sequences from other serotypes, conserved domains can be identified between the proteins. One of the conserved domains is positioned at a.a. 11-15 (LTGQS) and is probably important for the function of pTP since deletion of a.a. 1-18 (the protein encoded by the open-reading frame) does not encode a functional pTP.

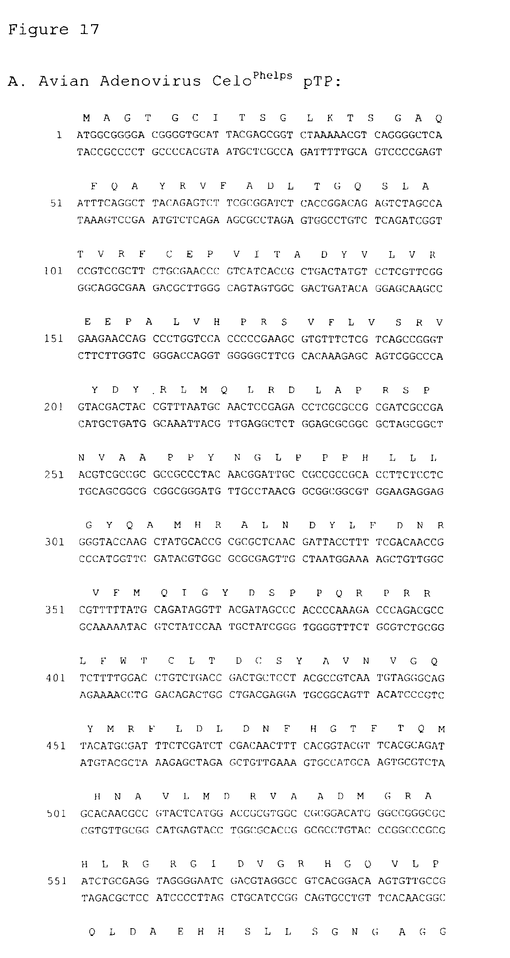

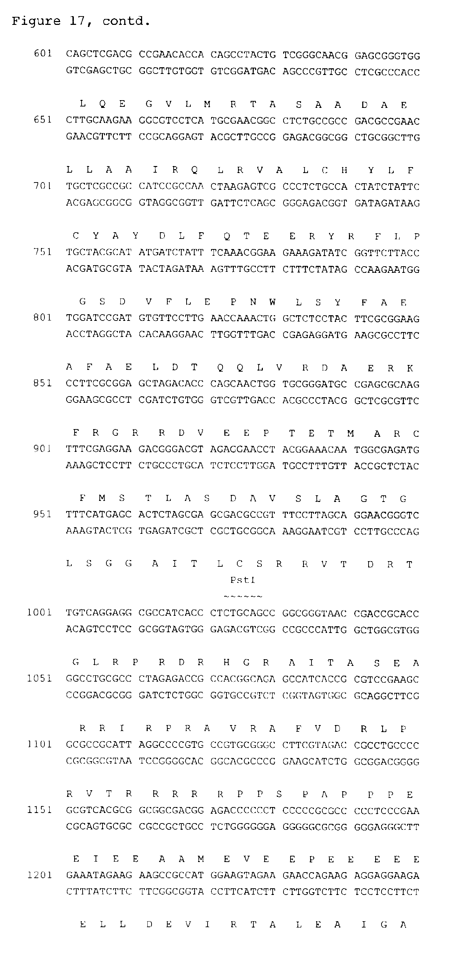

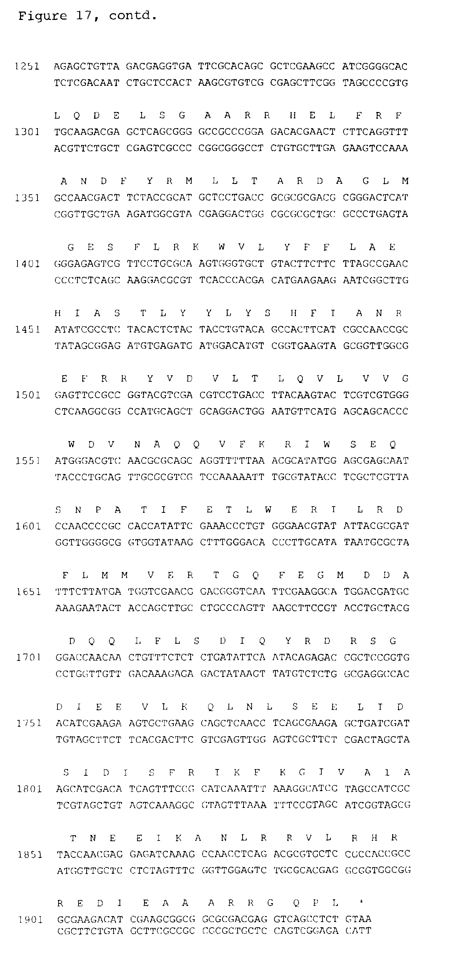

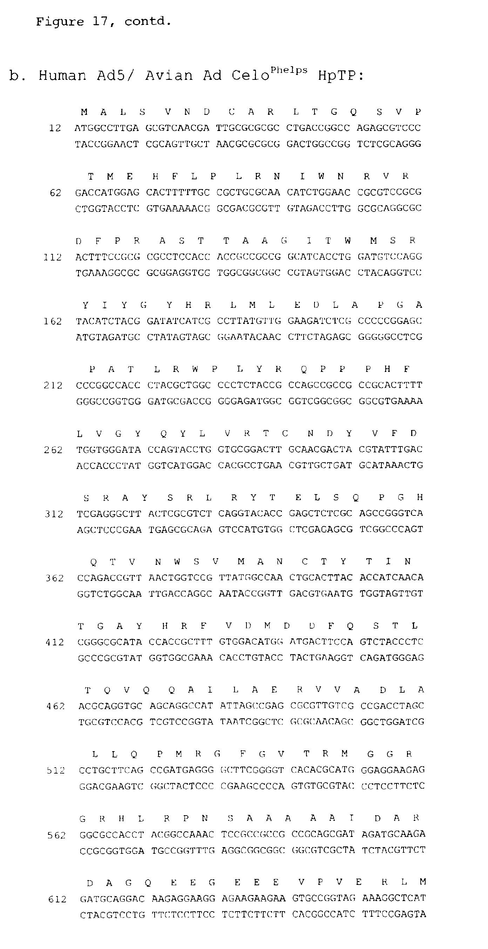

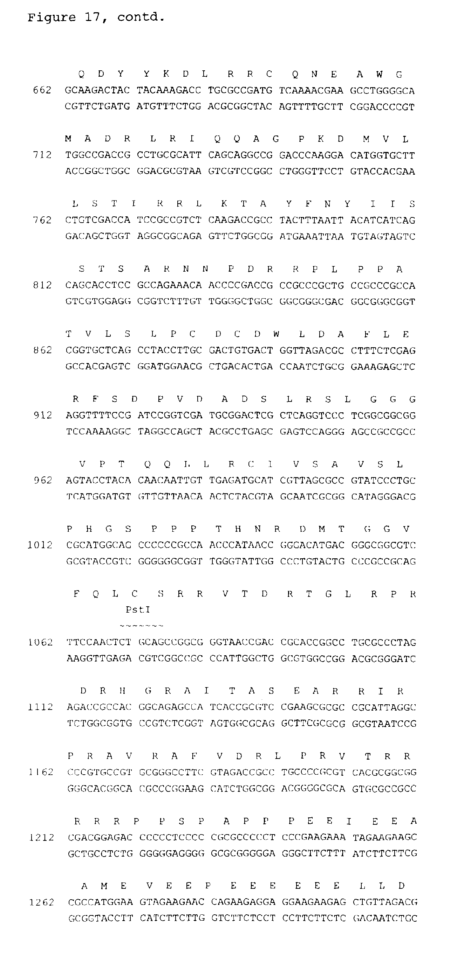

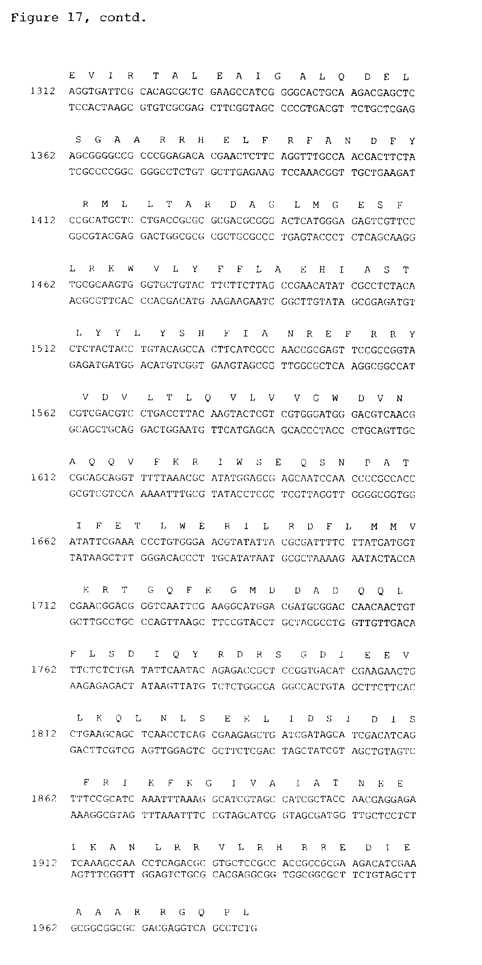

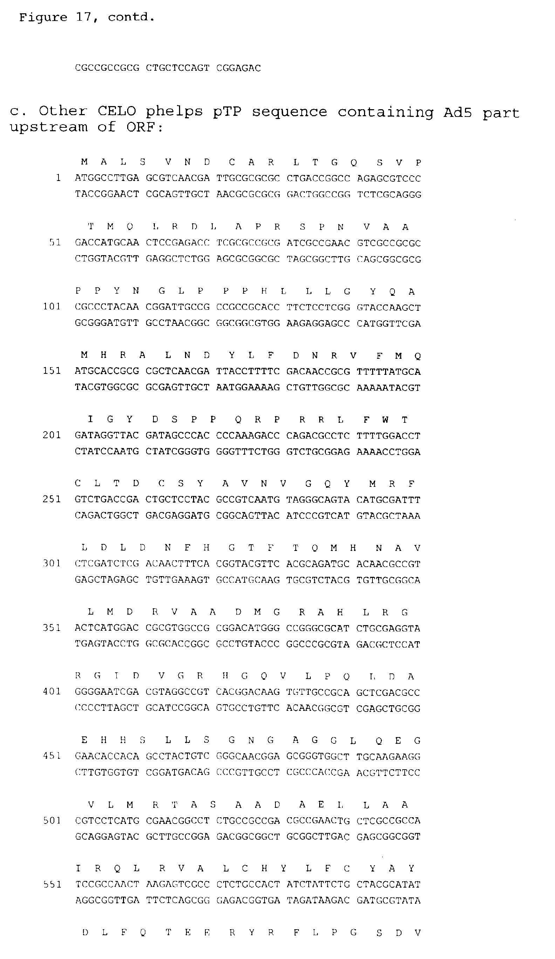

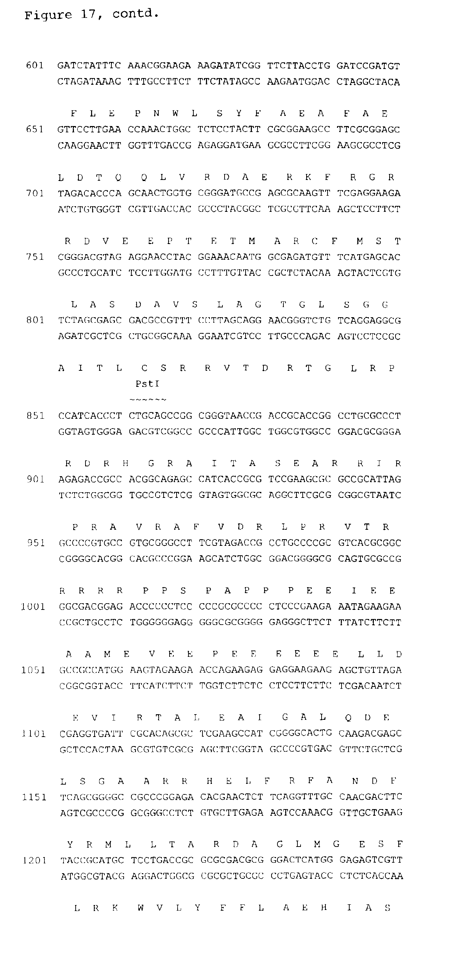

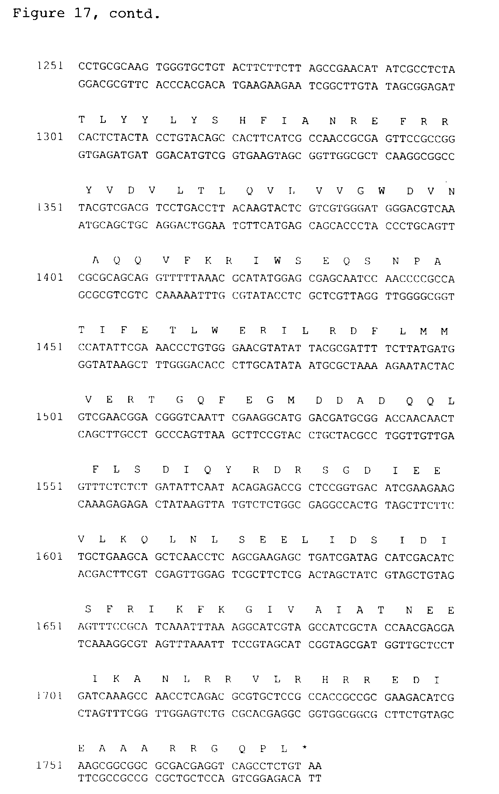

For CELO^{PHELPS} pTP the open reading frame that was first identified is located at nt 10269-11996 (1728 bp see also figure 17, genbank ID U46933). Due to the fact that the known human pTP pre-mRNA undergo splicing it is possible that CELO^{PHELPS} pTP pre-mRNA is also spliced. To determine if CELO^{PHELPS} pTP shares homologous domains upstream of the open reading frame, the upstream genomic sequence was translated from the start codon of the open reading frame to the first stop codon (nts 12249-12251) that was encountered. This sequence was aligned against the pTP sequence of several other known adenovirus serotypes. From the alignment it appeared that CELO^{PHELPS} pTP also contains the conserved LTGQS domain upstream of the first identified open reading frame. Since translation upstream of the start codon first gives a stop instead of a start codon it is likely that the CELO^{PHELPS} pTP is spliced to enclose the LTGQS domain.

The sequence from the stop codon (nts 12249-12251) to the start codon of the open reading frame (nts 11994-11996) was screened for putative splice acceptor sites using a splice prediction database (http://www.fruitfly.org/seq_tools/splice.html). It appeared that there were two putative splice acceptor sites between 12019 and nt 12020 (predicted value 48%) and between nt 12205 and nt 12206 (predicted value 88%). The splice acceptor site at position 12020 is not a likely candidate because if splicing would occur at that position the LTGQS domain would be lost. Based on these data the splice acceptor site for CELO^{PHELPS} pTP is located between nt 12205 and nt 12206. There were no splice donor sites identified in the screened sequence.

To determine the position of the start codon we compared the genomic organisation of human Adenovirus type 5 and Avian Adenovirus CELO^{PHELPS}. Determination of the start codon by sequence homology turned out to be useless since this open reading frame of Ad5 is not conserved in CELO^{PHELPS}. So we determined the position of the pTP start codon relative to the start codon of the structural protein L3 encoding open reading frame. For Ad5 the start codon is located 36 bp upstream of the L3 open reading frame (nt 14156-15871). To make sure that the Ad5 L3 open reading frame is homologous to the L3 open reading frame determined for CELO^{PHELPS} (nt 15110-16657) an alignment of the translation product of both open reading frames has been performed using the Blast engine. It appeared that the translation products of both open reading frames have 42% identity and are 54% positive. Thus both open reading frames are similar. Upstream of the start codon of the CELO^{PHELPS} encoded L3 open reading frame a number of putative CELO^{PHELPS}, pTP open reading frames have been identified. The most likely candidate was the open reading frame 23 bp upstream of the start codon of the L3 open reading frame since this open reading frame starts with ATG and encoded for the amino-acids methionine and alanine as in Ad5 pTP. This open reading frame (nt 14877-15086) was used to screen for putative splice donor sites (we used nt 14877-15119). It appeared that there was one putative splice donor site within this open reading frame (predicted value 99 %) between nt 15079 and nt 15080. Nucleotide seven at the splice donor site forms together with two nucleotides at the splice acceptor site one codon which results in the formation of an in frame pTP of 647 amino acids. In the Ad5 pTP protein, serine 580 is responsible for dCMP binding. For the CELO^{PHELPS} pTP protein, the amino acid that binds the first residue of the new DNA strand was mapped at serine 582. The protease sensitive sites iTPl, iTP2 and TP have been mapped between amino acids 188 and 189, 195 and 196, and 337 and 338, respectively.

A sequence for the CELO^{PHELPS} pTP protein is given in figure 17.

To enable replication of the chimaeric adenoviral vector of the invention it is not only necessary to provide a pTP protein capable of binding to the origin. An adenovirus polymerase capable of interacting with said pTP is also required. Furthermore, to enhance the replication efficiency said polymerase and/or said pTP protein should be able to interact with other proteins. For instance, proteins capable of binding to the adenovirus ITR that stimulate the replication.

In another aspect the invention provides a kit of parts capable of starting replication of a chimaeric adenoviral vector according to the invention, comprising a functional precursor terminal protein capable of binding to a site for protein primed replication initiation on said adenoviral vector and an adenovirus polymerase capable of binding to said precursor terminal protein. Preferably, at least a nucleotide binding part of said precursor terminal protein does not originate from the wild type adenovirus from which said packaging signal originates. By changing the nucleotide binding properties of said pTP one can generate a pTP with a specificity for a guanine, a cytosine, a thymidine and/or an adenine. Depending on the adenovirus from which said packaging originates one can choose the desired pTP or pTPs. For instance when the packaging signal originates from an adenovirus with cytosine at the indicated positions in the replication initiation origin a pTP can be used that is not capable of recognizing said cytosine but instead is capable of recognizing a guanine, a thymidine and/or an adenine. Preferably, said precursor terminal protein is also capable of binding to Oct-1. Thereby allowing a more efficient generation of chimaeric adenovirus vectors for use in humans. For the same reason said polymerase is, preferably, also capable of binding to NFI.

In one embodiment said polymerase is a fusion protein comprising polymerase activity from a first adenovirus and comprising precursor terminal protein binding activity from a second adenovirus, wherein said first adenovirus comprises a site for protein primed replication initiation not present in said second adenovirus. Thereby allowing the use of an unmodified pTP in combination with a modified polymerase. This embodiment is useful for increasing the efficiency of production of the chimearic vector in some embodiments of the chimaeric vector of the invention.

In another embodiment the precursor terminal protein is a fusion protein comprising polymerase binding activity from a first adenovirus and comprising binding activity for a site for protein primed replication initiation from a second adenovirus, wherein said first adenovirus comprises a site for protein primed replication initiation not present in said second adenovirus. This embodiment is useful for increasing the efficiency of production of the chimearic vector in some embodiments of the chimaeric vector of the invention.

In another aspect the invention provides a method for producing a rescue defective adenoviral gene delivery vehicle comprising contacting a chimaeric adenoviral vector according to the invention with a kit of parts according to the invention under conditions allowing for replication and packaging said adenoviral gene delivery vehicle. In one embodiment of the invention condition allowing for replication and packaging said adenoviral gene delivery vehicle are found in a cell. In a preferred embodiment said cell is permissive for replication and packaging of the wild type adenovirus from which said packaging signal originates. Preferably, said cell is a human cell.

In another aspect the invention provides a method for providing at least a partial helper function for the production of an adeno-associated virus and/or an adeno-associated virus vector in a cell comprising providing said cell with a chimaeric adenoviral vector according to the invention. Said chimaeric adenoviral vector can then provide at least part of the helper function required for the production of said adeno-associated virus and/or adeno-associated virus vectors. Adeno-associated virus replication in a cell requires the presence in said cell of at least some proteins of a helper virus. Currently recognized helper viruses for adeno-associated virus are members of the adenoviruses, the herpes viruses and vaccinia viruses. In a preferred embodiment the helper function provided by said chimaeric adenoviral vector comprises products of the E1, E2, E4 and/or the VA region of an adenovirus. For the production of an adeno-associated virus vector, also the products encoded by the rep-gene and the cap gene are required. Said products may be provided to said cell in the form of nucleic acid encoding said products. Said nucleic acid may be provided to said cell with a chimaeric adenoviral vector of the invention. An advantage of using a chimaeric adenoviral vector of the invention to provide at least in part the helper function for adeno-associated virus and/or adeno-associated virus vector production is that said chimaeric adenoviral vector is replication defective in said cell and thus cannot compete for resources with the adeno-associated virus and/or adeno-associated virus vector in the cell. Another advantage is that said chimaeric adenoviral vector is replication defective in said cell and thus is not efficiently packaged into adenovirus particles that may be formed during the production, thus obviating difficult purification procedures to eliminate the adenovirus from the adeno-associated virus preparation.

In another aspect the invention provides a method for the production of a minimal adenovirus vector without essentially also producing other adenovirus vectors and/or virus comprising providing a cell with said minimal adenovirus vector and a chimaeric adenoviral vector according to the invention. In a preferred embodiment said minimal adenovirus vector comprises a functional site for protein primed replication initiation originating from said wild type virus said packaging signal originates from.

A used herein the term "minimal" when referring to an adenovirus vector, means that the vector comprises a left and a right ITR and a packaging signal, preferably in the vicinity of an ITR. The minimal vector may of course comprise additional genetic information such as one or more gene of interest. The minimal vector may further comprise other adenovirus sequences as long as said sequences are essentially not expressed in the target cell and/or virus producing cell. A rdAd that is also a minimal Ad vector therefore comprises a packaging signal of an adenovirus and at least one ITR, preferably at least two ITR, comprising a site for protein primed replication initiation not present in the wild type adenovirus from which said packaging signal originates, thereby essentially disabling replication of the minimal vector under conditions that would allow replication in the case of the minimal vector having a site for protein primed replication initiation present in the wild type adenovirus from which said packaging signal originates.

### Examples

### Example 1

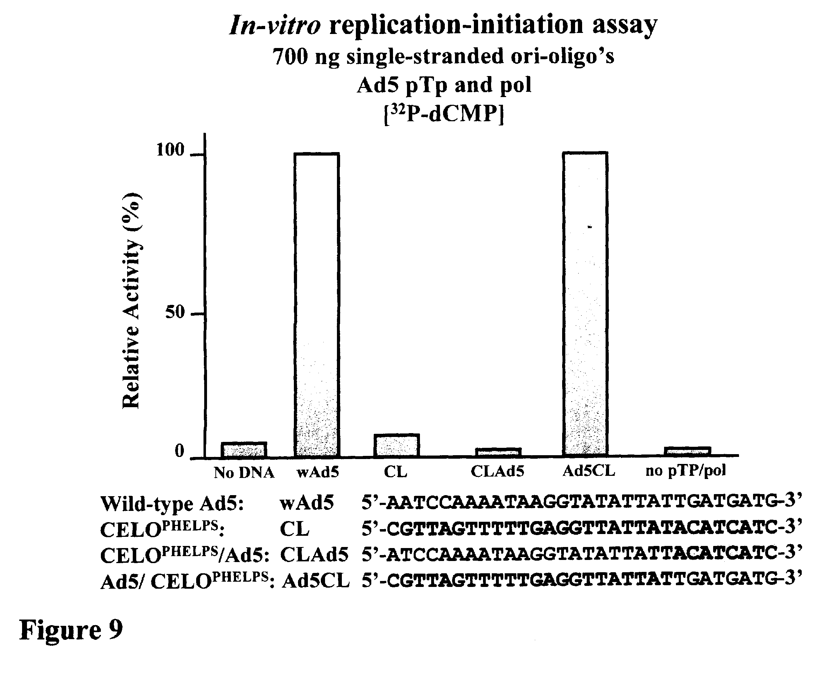

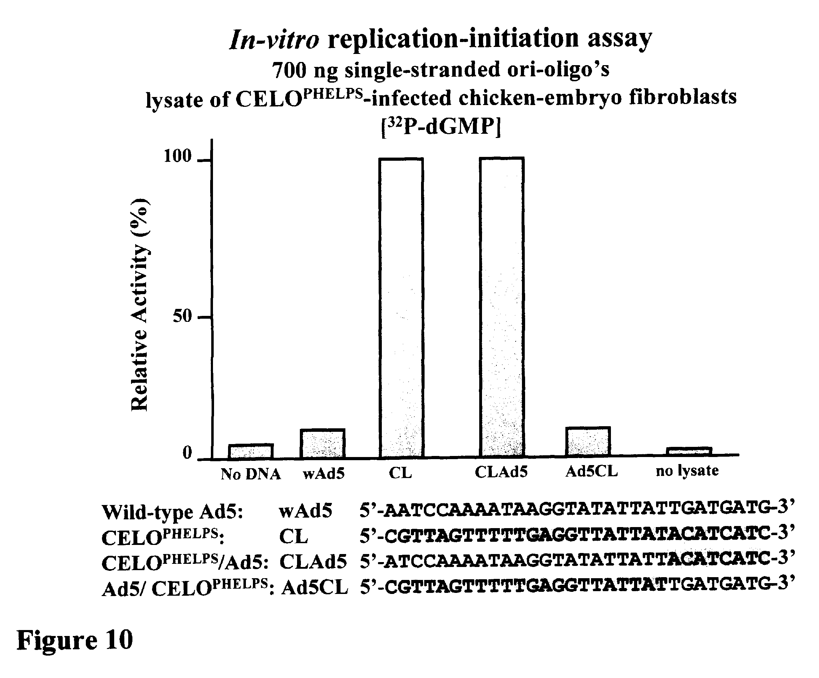

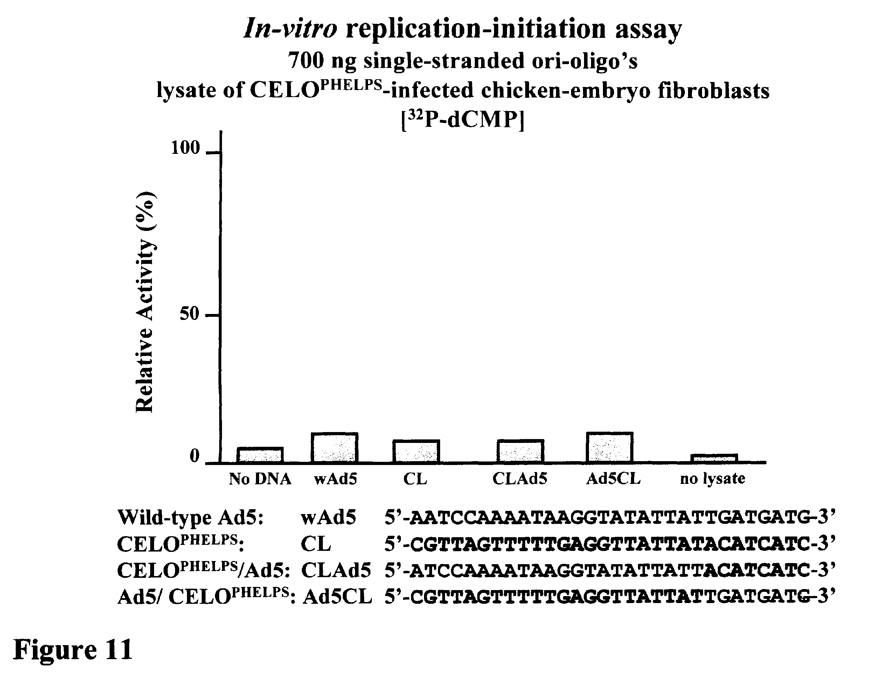

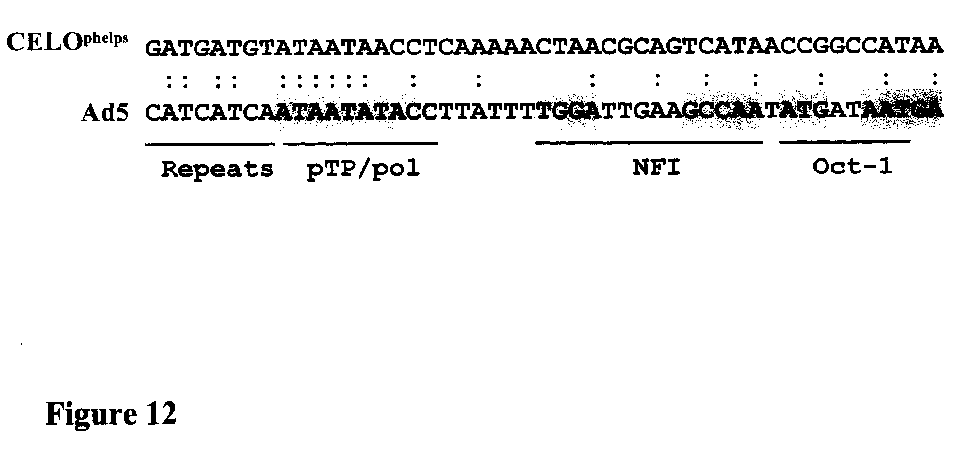

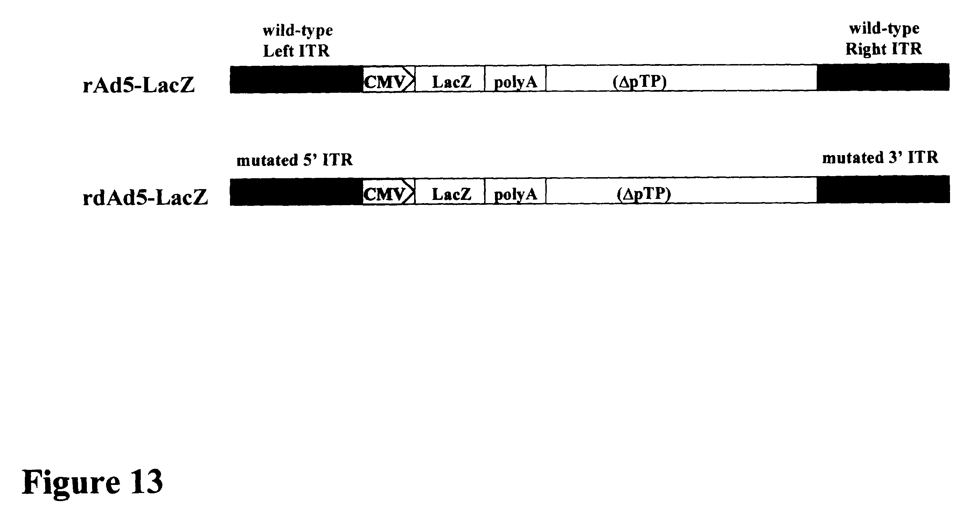

Strikingly, the start of the CELO^{PHELPS} origin of replication closely resembles an Ad5 origin, but contains C's at the position of G1, G4, and G7 in the Ad5 sequence. This implies that CELO^{PHELPS} pTP, but not Ad5 pTP, can initiate the replication of an Ad5 origin in which the guanines at positions one, three, and seven have been replaced by cytosines. This has been confirmed by *in-vitro* replication analyses. First, we tested whether the Ad5 replication machinery could initiate replication of the CELO^{PHELPS} origin of replication. This was tested by means of an *in-vitro* replication assay as described by King and van der Vliet (King and van der Vliet, 1994). In this assay, initiation intermediates are characterized using highly purified pTP-pol and TP-DNA or single-stranded oligonucleotides as templates. Upon the addition of a radiolabeled dNTP to the reaction mixture, formation of a labeled pTP-dNTP complex can be detected by SDS-polyacrylamide gelectrophoresis and autoradiography. As shown in Fig. 9 , replication of a 30-nt single-stranded (ss) Ad5-ori oligo was efficiently initiated, as judged by the formation of labeled pTP-dCMP complexes. In contrast, we could hardly detect formation of this complex when we used a 30-nt ss CELO^{PHELPS} ori-oligo as the template. We also tested two hybrid ori-oligo's (Fig. 9 ), one (CLAd5) carrying the first eight nt of the very end of the CELO^{PHELPS} ITR, followed by nt 9 to 30 of the Ad5 sequence, and the other (Ad5CL) *vice versa.* In the replication assay with the hybrid Ad5CL-ori oligo as the template, formation of labeled pTP-dCMP complexes occured to the same level as that observed with the wild-type Ad5-ori oligo. However, when we used the CLAd5-ori oligo's, we could barely detect formation of pTP-dCMP complexes. These results can be explained by the fact that the Ad5 pTP is only able to initiate replication by formation of pTP-dCMP complexes: Ad5 pTP can not form complexes with dATP, dGTP, or dTTP (King and van der Vliet, 1994). Thus, the Ad5 replication machinery can not initiate replication of DNA templates that depend on formation of Ad5-pTP-dGMP complexes, as is the case for the CELO^{PHELPS} ori-oligo and the CLAd5 hybrid ori-oligo. Next, we repeated the *in-vitro* replication assay using the same set of ori-oligo's, but with radiolabeled dGMP, and a protein-lysate of CELO^{PHELPS}-infected chicken-embryo fibroblasts. This lysate should contain proteins (pTP and pol) that are involved in replication of the CELO^{PHELPS} genome. In this experiment, we found that the CELO^{PHELPS}- and the hybrid CLAd5-ori oligo's allowed formation of labeled CELO^{PHELPS}-pTP-dGMP complexes, but not the wild-type Ad5- or Ad5CL-ori oligo's (Fig. 10). When we used radiolabeled dCMP, none of the above mentioned ori-oligo's enabled complex-formation between the labeled dCMP and CELO^{PHELPS}-pTP (Fig. 11). From these results, we concluded that CELO^{PHELPS}-pTP can form complexes with dGMP, but not with dCMP.
Various factors may contribute to the fact that CELO^{PHELPS} can not use cells of human origin for the production of progeny virus, including the lack of an essential host cellular receptor (such as CAR for Ad5 (Bergelson *et al.,* 1997)). In addition, it seems likely that CELO^{PHELPS} needs, like Ad5, specific cellular transcription factors for optimal replication. Although such factors have not been identified for CELO^{PHELPS} yet, it may be that the binding of such cellular proteins determines the host-range of CELO^{PHELPS}. A comparison of the first 50 terminal base pairs of the avian adenovirus CELO (phelps strain) and the human adenovirus serotype 5 (Fig.12) indicates the absence of recognition signals for cellular transcription factors NFI and NFIII (Oct-1) in the CELO^{PHELPS} origin of replication. Thus, if CELO^{PHELPS} uses cellular transcription factors during replication of its genome, it could be that they are not present in human cells, including helper cell lines 293, 911, and PER.C6. Therefore, instead of employing the complete CELO^{PHELPS} ITRs in conjunction with the Ad5 genes, we have constructed hybrid Ads, which we named rdAd (rescue-defective Ad). A model rdAd, rdAd-LacZ, is deleted of E3 and carries the E. coli LacZ gene at the position of E1, and contains modified ITRs as depicted in Fig. 13. rdAd5-LacZ was generated using the method developed by He and co-workers (He et al., 1998). For this system several plasmids have been constructed. To generate rdAds we modified the pShuttle plasmid (Fig. **13?**). In this plasmid, the left ITR can be removed by digestion with XmnI and KpnI.
The hybrid left ITR was synthesised by PCR using the forward primer 5'-CGAATTTTTTCTTAATTAAGATGATGTATAATATACCTTATTTTG-3' and the reverse primer 5'-CGGTACCATCGATAGTATTACGCGCTATGAG-3' on pShuttle as template. The resulting 376 bp PCR fragment was digested with XmnI and KpnI and ligated into the 6.3 kb XmnI-KpnI fragment of pShuttle. This construct now contains a unique ClaI site in contrast to pShuttle.
For the synthesis of the hybrid right ITR the forward primer 5'-CCCTAGGCAAAATAGCACCCTCC-3' and reverse primer 5'-TGGATCCCGATCGTTAATTAAGATGATGTATAATATACC-3' have been used in a PCR on pShuttle as template. The 497 bp PCR fragment was digested with BamHI and AvrII and ligated into the 6.1 kb BamHI-AvrII pShuttle fragment containing the previous described hybrid left ITR.
This plasmid can be used to clone the gene of interest, and after recombination against e.g. pAdEasyI will generate rdAd genomes.

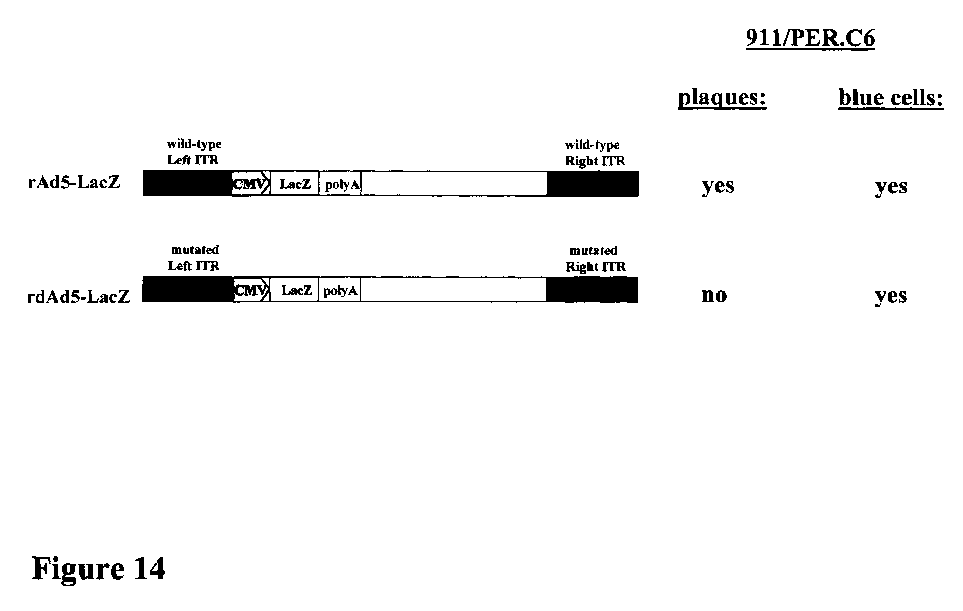

The model rdAd, rdAd5-LacZ was constructed as following. Plasmid pCH110 (Pharmacia), was digested with TthIII I, blunted with Klenow polymerase, and digested with BamHI. The 4.2 kb fragment carrying the eukaryotic SV40 and prokaryotic gpt promoter, the LacZ coding region, and the SV40 polyadenylation signal was cloned in the EcoRV and BglII sites of the pShuttle plasmid carrying the hybrid ITRs. The resulting plasmid was linearized with PmeI, and by recombination with plasmid AdEasy I (as described by He *et al.,* 1998) rdAd5-LacZ DNA was generated. Replication of rdAd5-LacZ was monitored by transfection of bacterial rdAd5-LacZ DNA into 911 or PER.C6 cells. As a control, we transfected rAd5-LacZ DNA, carrying wild-type Ad5 ITRs (Fig. 14). The transfected monolayers were either stained with X-gal two days post-tranfection to screen for blue cells, or were embedded in agar-medium and screened for plaque-formation. As shown in Fig. 14, transfection of rAd5-LacZ DNA into 911 or PER.C6 cells resulted in blue cells, and in formation of plaques as a result of replication and subsequent generation of progeny virus. In contrast, although we could detect blue cells after X-gal staining of 911 or PER.C6 cells transfected with rdAd5-LacZ DNA, plaques could not be detected. Most likely, this is due to the fact that the rdAd5-LacZ DNA had not been replicated, disabling generation of progeny virus and plaque-formation. The absence of replication of rdAd5-LacZ DNA was confirmed by southern analyses of hirt-extracted DNA from PER.C6 cells transfected with rdAd5-LacZ DNA. The extracted extra-chromosomal DNA was digested with restriction enzyme *Dpn*I, which only cuts bacterially methylated DNA, or with DpnII, which does not cut bacterially methylated DNA. Subsequently, the DNA was size-fractionated on a 1% -agarose gel, and transferred to a nylon filter. The filtered was then hybridized to a radiolabeled LacZ probe. We detected a specific signal in the lane containing *Dpn*II-digested DNA from the rAd5-LacZ-transfected PER.C6 cells (not shown). This indicated the presence of *the novo* synthesized (replicated) rAd5-LacZ DNA. In contrast, the diagnostic signal was not visible in the lane containing *Dpn*II-digested DNA from the rdAd5-LacZ-transfected PER.C6 cells (not shown). Apparently, the transfected rdAd5-LacZ DNA was not replicated in the PER.C6 cells. We could, however, detect the presence of *Dpn*II-resistant, non-replicated input rdAd5-LacZ vector DNA (not shown), which demonstrates that rdAd5-LacZ vector DNA was present in the PER.C6 cells. From these results we concluded that rdAd5 vectors, carrying modified ITRs as in rdAd5-LacZ, can not be replicated by the Ad5 replication machinery. We expect that this is also true for all other currently known human Ads, since all known Ad genome-ends, with the exception of CELO^{PHELPS}, start with a C residue (Van Ormondt and Galibert, 1984).

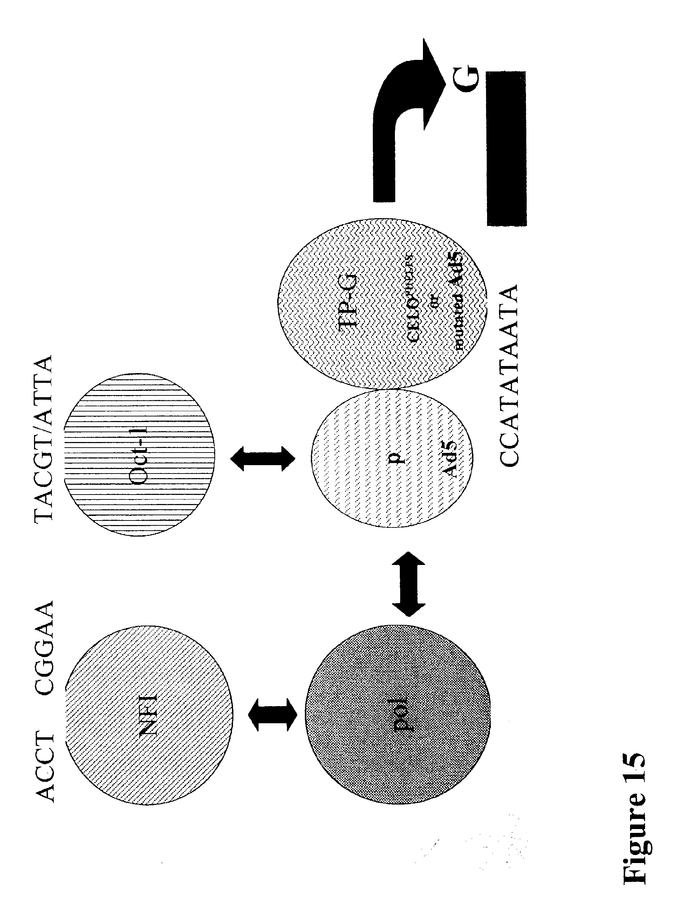

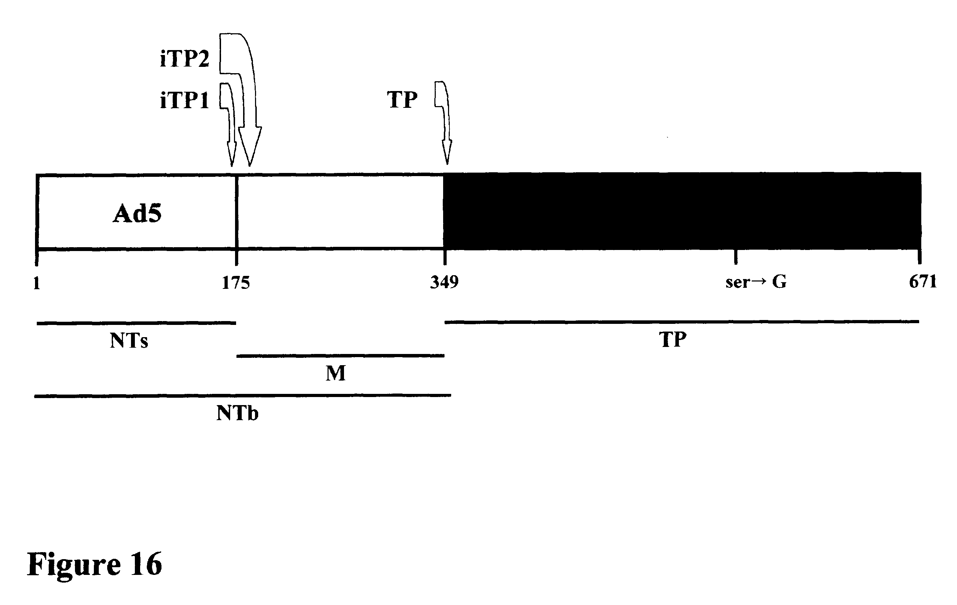

In order to propagate rdAd vectors, we constructed PER.C6-based cell lines expressing a pTP that complexes with dGMP (designated pTP-G). The most straightforward strategy would have been to generate 911- or PER.C6-based helper cells that express the CELO^{PHELPS} pTP gene. However, from many years of intensive research on the mechanism of Ad5 replication (for a comprehensive review, see Van der Vliet, 1995) it is now known that efficient replication initiation of Ad5 requires that pTP interacts with the Ad5 polymerase (pol) and with a cellular transcription factor, Oct-1 (Fig. 4). Mutations in the coding region of Ad5 pTP that interfere with pol or Oct-1 binding greatly affect replication. Therefore, we have designed several strategies to generate a pTP that binds to dGMP, and not to dCMP, and also functionally interacts with pol and Oct-1 (Fig. 15). For this purpose, we have constructed several Ad5 pTP mutants with altered nucleoside specificity, and a hybrid pTP that carries the precursor part of Ad5 pTP, and the TP part of the CELO^{PHELPS} pTP, which we named hpTP-G (Fig. 16). This hybrid pTP was constructed since is has been demonstrated that the precursor part of pTP is responsible for the interaction with pol (Hay, 1995; Webster *et al.,* 1997) and Oct-1 (Botting and Hay, 1999), while a serine in the TP part (serine 580 in the case of Ad5) is responsible for the binding of the first residue (Fig. 5, panel C).

For the construction of the hybrid pTP (hpTP) a fusion between the Ad5 `pre' (genomic nt 9541-10589 and 14111-14119) and CELO^{PHELPS} 'TP' (genomic nt 10269-11191) was generated. Since the maturation of hpTP to TP is dependend on the action of the human adenovirus type 5 encoded proteases, the protease sensitive site TP in hpTP should be of an Ad5 origin (Fig. 16). Therefore, we decided to include the Ad5 encoded VFQL sequence instead of the CELO^{PHELPS} encoded AITL to the N-terminus of the hybrid TP.

Immediately downstream of the AITL codons in CELO^{PHELPS} pTP a PstI site is present. We used this site to combine an Ad5 'pre' encoding PCR fragment with a CELO^{PHELPS} TP encoding PCR fragment.

For the synthesis of the Ad5 `pre` PCR fragment the 5'-TAAAGCTTGCCACCATGGCCTTGAGCGTCAACGA-3' forward primer and the reversed 5'-GACTGCAGAGTTGGAAGACGCCGCCCGT-3' primer were used in a PCR with pVAC-pTP as template. The forward primer also contains an optimised Kozak sequence downstream of the start codon. For the synthesis of the TP PCR fragment the forward primer 5'-TAAAGCTTGCCACCATGGCCTTGAGCGTCAACGA-3' and the reversed primer 5'-TTGGATCCTTACAGAGGCTGACCTCGTCG-3' have been used in a PCR using CELO^{PHELPS} genomic DNA as the template.

The 'pre' PCR fragment digested with HindIII and PstI was ligated with the PstI-BamHI digest of the TP PCR product in the 2.7 kb BamHI-HindIII pCDNA3.1+ fragment. The sequence of hpTP is shown in Fig. 17. Full length CELO^{PHELPS} pTP was synthesized by PCR. The pTP-G mutants of Ad5 pTP were generated as following. Random mutants of Ad5 pTP were generated by error-prone PCR. Mutants with altered nucleoside specificity were selected by co-transfection of rdAd-LacZ DNA and a pool/library of (linearized) plasmids carrying Ad5 pTP mutants into 911 helper cells. In this manner, the Ad5 pTP in rdAd-LacZ could be replaced by a mutant pTP *via* homologous recombination. Only the recombinants that obtained a mutant pTP capable of binding to dGMP, i.e. pTP-G mutants, were expected to form plaques. Virus from individual plaques was amplified, and the mutated pTP sequence was analyzed. The pTP-G-encoding DNA, isolated from a rdAd-LacZ/pTP-G virus that grew to relatively high titers, was cloned in a mammalian expression vector, as was hpTP-G. These plasmids were used for the generation of 911-(h)pTP-G and PER.C6-(h)pTP-G.

Replication of rdAd5-LacZ on 911-pTP-G and PER.C6-pTP-G was assayed by plaque-formation and southern analyses as described above. Transfection of bacterial rdAd5-LacZ DNA into the pTP-G helper cells resulted in blue cells, and in formation of plaques, whereas transfection with rAd5-LacZ DNA only resulted in blue cells. Replication of rdAd5-LacZ in 911-pTP-G and PER.C6-pTP-G was confirmed by southern analysis, similar to those described above (not shown).

Subsequently, we determined the yields of rdAd-LacZ obtained from 911-pTP-G and PER.C6-pTP-G, and compared them to the yields of rAd-LacZ produced by the parental 911 and PER.C6 cells. We found the yields to be similar (data not shown), and therefore we concluded that rdAd5 vectors, carrying modified ITRs as in rdAd5-LacZ, can be propagated on 911-pTP-G and PER.C6-pTP-G.

### Example 3 Some applications of the invention

### 3.1 Rescue-defective Ad vectors

The use of rdAds provides an important safety feature, namely the fact that such recombinant vectors can not be rescued by wild-type Ads, thus preventing spreading of the viral vector. Therefore, the rdAd technology is useful for all *in vivo* studies using recombinant Ad vectors. The technology is particularly useful for the treatment of cystic fibrosis patients with recombinant vectors carrying the cystic fibrosis transmembrane conductance regulator (CFTR) gene, since the recombinant Ad is administered to the airways of the patients, which is the primary site of infection for human Ads. Thus, in this example, the risk of rescue of the CFTR vector by wild-type Ads is most prominent. The use of a rdAd-based CFTR vector would eliminate the risk of rescue and spreading of the recombinant virus.

### 3.2 "Gutless" Ad vectors

Ad vectors are among the most efficient gene-transfer vehicles for both *in vitro* and *in vivo* delivery, but the utilization of current Ad vectors for many gene therapy applications is limited by the transient nature of transgene expression obtained by these vectors. Several factors have been shown to contribute to and modulate the duration of Ad-mediated expression and the immunogenicity of these vectors, including "leaky" viral expression and the transgene that is delivered. The development of Ad vectors that are deleted in all viral protein-coding sequences, so-called "gutless Ads" (gAd), offers the prospect of a potentially safer, less immunogenic vector with an insert capacity of up to 37 kb. gAds can be propagated with the use of a helper virus that supplies the Ad proteins required for replication and packaging. Currently, the cre/lox-helper system is the preferred method for production of gAds (Parks, et al 1996). In this system, an El-deleted Ad is used with bacteriophage P1 *loxP* recombination sites flanking the packaging signal ("floxed"; reviewed by Morsy and Caskey, 1999). Upon infection of the 293 helper cells that express the *cre* recombinase, the packaging signal is deleted from the helper virus DNA, thus creating an Ad genome that can not be packaged into new virus particles. This Ad DNA can serve, however, as a helper for the gAd, by delivering proteins for replication and packaging.

A drawback of the current cre/lox-system is that the produced virus batch does not consist for 100% of gAds: depending on the production scale, the virus batch may contain upto 0.1% helper virus or replication-competent adenovirus (RCA). There are several explanations for these contaminations. Firstly, the helper DNA can recombine homologously with the Ad5 DNA that is present in the 293 helper cells (Fallaux *et al.,* 1998). This will generate RCA, in this particular case Ads that have regained a wild-type left ITR (packaging signal not floxed) and the Ad5 E1 region. Secondly, homologous recombination can occur between the left ITRs of the DNA of the helper virus and the gAd DNA, resulting in an El-deleted Ad with a wild-type left ITR. Thirdly, since the helper Ad DNA replicates upto many thousand copies per cell, and the helper-cell protein synthesis is switched-off during the late phase of the lytic production, the amount of cre present in each helper cell may be sub-optimal. Thus, many helper DNA molecules may escape from cre-mediated excision of the packaging signal, leaving Ad DNA molecules that can be packaged in progeny virus particles.

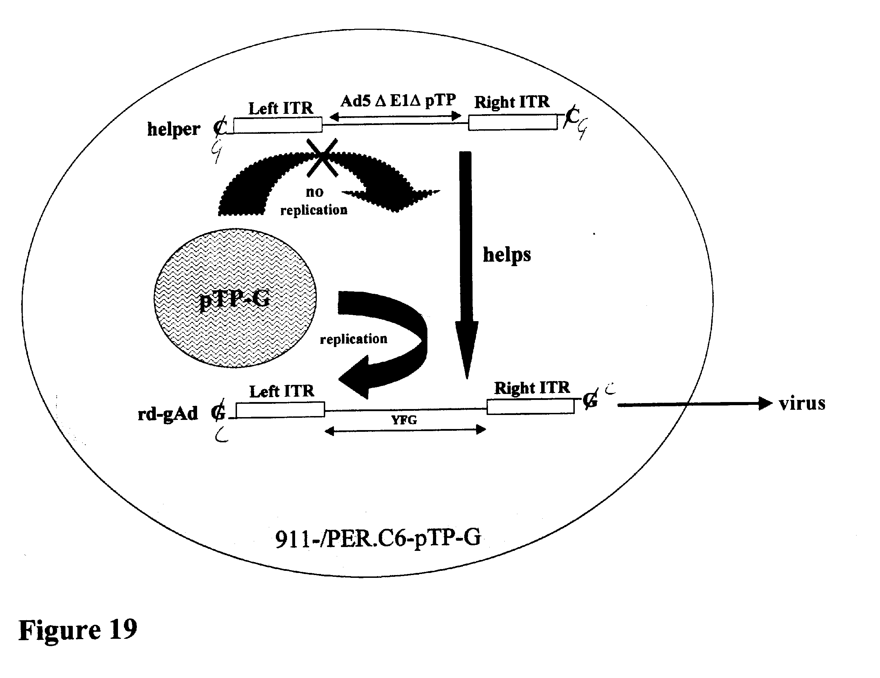

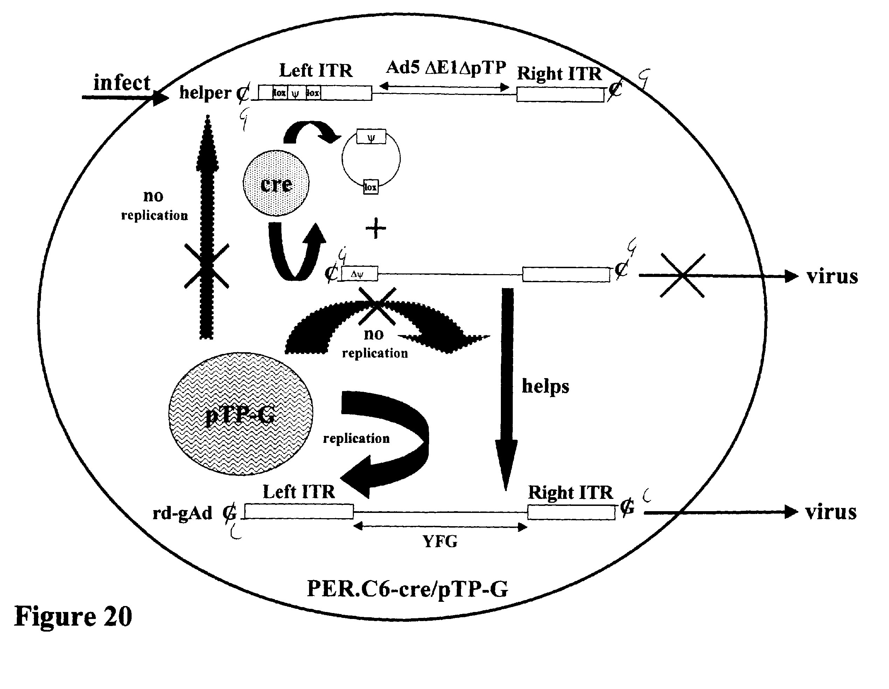

Our invention can also be used for the production of gAds. In this case, gAd production would rely on the use of a helper Ad that does not replicate in the 911-/PER.C6-pTP-G packaging cells (Fig. 19). Combination of our invention with the cre-lox technique, i.e. a rdAd helper virus with floxed packaging signal (Fig. 20) would drastically reduce the amount of helper-DNA molecules that must be attacked by the cre recombinase. This could further reduce or completely eliminate the helper virus contamination in gAd batches. In addition, the use of PER.C6-pTP-G would also eliminate the formation of RCA through homologous recombination. For this application, it is desirable to provide expression of structural protein IX *in trans,* as it has been shown that expression of adenovirus protein IX is dependent on replication (Vales and Darell, 1989).

### 3.3 Adenoviral vectors as recombinant viral vaccines

Adenoviruses can efficiently induce immunity in the lung following single enteric delivery. They can also be engineered to express a number of heterologous proteins *in* *vitro* and *in vivo.* Recombinant Ads offer a number of favorable features to develop new vaccines (reviewed by Imler, 1995): they are not associated with severe pathologies in humans (Horwitz, 1990); their genome has been extensively studied and the complete DNA sequence of some serotypes is known (Chroboczek *et al.,* 1992); methods to construct rAds are well established; and they can be administered orally (Top *et al.,* 1971a; Top *et al.,* 1971b; Schwartz *et al.,* 1974). The popularity of Ads as a recombinant viral vector is largely due to the successful and safe immunization of millions of US military recruits with enteric coated Ad4 and Ad7 as a prevention against acute respiratory disease (ARD) outbreaks (Top *et al.,* 1971a).

Following these first trials, a number of rAds have been constructed and tested in animals. The choice of the animal model is mostly determined by the susceptibility to challenge with the infectious agent against which protection is sought, such as the cow for vesicular stomatitis virus (Prevec et *al.,* 1989), the cottontop tamarin for Epstein Barr Virus (Ragot *et al.,* 1993), the chimpanzee for human immunodeficiency virus (Natuk *et al.,* 1993), and the ferret for respiratory syncitial virus (Hsu *et al.,* 1994). The majority of the animal trials employed replication-competent rAds with the expression cassettes for the foreign antigen in place of the E3 region, however, replication-defective rAds in which the expression cassette replaces the E1 region have been tested as well (reviewed by Imler, 1995).

Since human Ads have a restricted host range, and in most cases the animals used to test the vaccine are not permissive, the virus can not, or poorly, replicate. Notwithstanding, most protocols induced an immune response, independently of the antigen, the animal model, the vector-backbone (i.e. E1⁺/E3⁻, or E1⁻/E3^{-/+)}, or the route of administration (reviewed by Imler, 1995). Thus, replication of the Ad-based vaccine is no prerequisite for immunization. Indeed, Prevec and co-workers were able to induce good neutralizing responses with the same replication-competent virus and the same immunization protocol both in permissive and non-permissive species (Prevec *et al.,* 1989). The apparent efficiency of Ad-based vaccines in non- or semi-permissive species suggests proper antigen expression in the absence of viral replication. This is supported by several reports on the use of E1-deleted, replication-defective Ads: in all cases, high levels of antigen in a number of cell lines was obtained (Alkhatib and Briedis, 1988; Eloit *et al.,* 1990; Levrero *et al.,* 1991). In addition, immunization experiments using replication-defective Ads were able to induce an immune response and at least partial protection in animals.

Despite the encouraging results obtained with Ad-based vaccines so far in animals, the technology still has a number of potential disadvantages that block wide application in human patients. The first concern to be considered is safety, in particular the risk of spreading and dissemination of the recombinant virus. Although no such problems were encountered in the US army vaccination programs with wild-type Ad4 and Ad7 (Top *et al.,* 1971a; Top *et al.,* 1971b) and in adult trials with wild-type Ad1, Ad2, and Ad5 (Schwartz et *al.,* 1974), the outcome was different in trials designed to protect children from ARD. In trials that were performed in the 1960s using preparations and protocols similar to those used in the military recruits, dissemination of the vaccine to house-hold members was demonstrated (Mueller *et al.,* 1969).

Even when replication-defective Ads are used, the defective virus could be trans-complemented by a wild-type Ad. This creates a risk of co-dissemination of both viruses, in the patient and in the environment. To reduce this risk, Imler and co-workers (Imler *et al.,* 1995) have constructed rAds containing mutations in important cis-acting sequences that control the encapsidation of the viral genome in virions. Depending on the mutation, the packaging efficiency is more or less severely affected, and in the situation of a cell infected with both the Ad-vaccine and a wild-type Ad, the wild-type Ad-DNA is preferentially packaged, leading to a rapid dilution of the recombinant genome.

The construction of E1-deleted rdAd-based vaccines carrying mutated ITRs as described in the current invention would eliminate potential spreading and dissemination of the virus (due to replication), as well as rescue by wild-type Ads. Namely, the described mutation of the ITRs completely blocks replication, which prevents spreading or dissemination. In addition, trans-complementation by wild-type Ads is impossible, due to the fact that the replication machinery of all (currently known) human Ads is designed to incorporate a dCMP residue; initiation of replication of rdAds that carry the described mutated ITRs depends on incorporation of a dGMP residue.

### Brief description of the drawings

- Fig. 1: Schematic representation of the human adenovirus genome. Indicated are the positions of the early (E) and late (L) regions.
- Fig. 2: The adenovirus origin of replication. The first 53 nucleotides of the Ad5 ITR are shown. The terminal protein (TP) is covalently attached to the 5'dCMP residue through serine 580. Regions highly conserved in various serotypes are boxed. Binding sites for the precursor-terminal protein (pTP)-polymerase (pol) complex (pTP-pol) in the core origin and for cellular transcription factors NFI and NFIII/Oct-1 in the auxiliary region are indicated.
- Fig. 3: Sequence alignment of the first 50 terminal basepairs of adenoviruses. The consensus NFI and Oct-1 binding site are given under the sequences. Note that the HAd3 5'end is left out because this part has been scrambled due to a recombination event. The repeats and binding sites for pTP-pol, NFI and Oct-1 are indicated. Sequences were all obtained from the GenEMBL data bank. The letter added to Ad indicates the species: A = avian (CELO Ote strain), B = bovine, C = canine, E = equine, H = human, P = porcine, S = simian, T = tupaia, M = mouse. Dark boxes indicate identical basepositions in more than 50% of the sequences. Grey boxes indicate purine or pyrimidine identity in more than 50% of the sequences. - indicates inserted gap for optimal alignment. N = unknown base. (Dr. H. van Leeuwen, thesis).
- Fig. 4: Initiation of adenovirus replication. Shown are the replication factors assembled, protein - protein interactions, protein - DNA interactions, and the bent DNA structure. Abbreviations: pol: Ad polymerase; p: precursor-part of Ad terminal protein; TP: Ad terminal protein; NFI/III (Oct-1): cellular transcription factors nuclear factor I/III. The recognition sites for NFI/III and the pTP-pol complex are indicated.
- Fig. 5: Human Ad precursor terminal protein (pTP). Schematic diagram of pTP indicating the fragments that can be obtained by means of protease mapping and their relationship to the cleavage sites of the Ad2 protease. NTs is a fragment from amino acids 1 - 175, NTb from amino acids 1 - 349, M from amino acids 176 - 349, and TP from amino acids 350 - 671. Indicated is serine 580, which binds a dCMP residue.
- Fig. 6: Rescue of rAds by wild-type Ads. Since rAds are deleted of (at least) E1, they can not replicate autonomously in normal human cells, and their presence is therefore restrained to the infected cell. However, infection with a wild-type Ad can result in spreading of the rAd, as the wild-type Ad "rescues" the rAd by providing the E1 functions *in trans.* Indicated are schematic representations of a wild-type Ad genome, a rAd-LacZ genome, and the wild-type Ad pTP.
- Fig. 7: No rescue of rescue-defective Ads (rdAds) by wild-type Ads. Same as Fig. 5, but now the wild-type Ad can not rescue the rdAd, since the available wild-type pTP is not capable to initiate replication of the rdAd DNA. Thus, no progeny of the rdAd is produced, thereby preventing spreading of the rdAd.
- Fig. 8: Initiation of replication of the CELO^{PHELPS} genome. Same as Fig. 4, however, the first-to-be-build-in residue is a dGMP. Putative, though as yet unknown cellular transcription factors (TF) are indicated.
- Fig. 9: Wild-type Ad5 pTP does not allow initiation of replication of templates carrying C's instead of G's at nucleotide positions one, four, and seven. The formation of pTP-dCMP complexes was determined as described by King and Van der Vliet (King and Van der Vliet, 1994). Briefly, highly purified Ad5 pTP and pol were mixed with 700 ng of the indicated single-stranded oligo's in the presence of radio-labelled dCTP.
- Fig. 10: The CELO^{PHELPS} replication machinery allows initiation of replication *in vitro* of a human Ad5 template carrying C's instead of G's at nucleotide positions one, four, and seven. Same as Fig. 9, but using a protein lysate of CELO^{PHELPS}-infected chicken-embryo fibroblasts, and using radio-labelled dGTP.
- Fig. 11: The CELO^{PHELPS} pTP does not allow initiation of replication of templates carrying G's instead of C's at nucleotide positions one, four, and seven. Same as Fig. 10, but using radio-labeled dCTP.
- Fig. 12: Sequence alignment of the first 50 terminal basepairs of the avian adenovirus CELO (Phelps strain) and the human adenovirus serotype 5 (Ad5). The positions of the repeat region, and binding sites for pTP-pol, NFI and Oct-1 are indicated. Note that the recognition signals for NFI and NFIII/Oct-1 are absent in the CELO^{PHELPS} sequence.
- Fig. 13: Schematic representation of a rdAd. Indicated are the modified ITRs of a model rdAd (rdAd5-LacZ).
- Fig. 14: rdAd5-LacZ DNA does not replicate in 911 or PER.C6 cells. 10 µg rAd5-LacZ or rdAd5-LacZ DNA was transfected into sub-confluent 911 or PER.C6 cells in 6-well plates (two wells for each transfection). One well was overlayed with agar-containing culture medium, while the other well received normal liquid medium. After seven days, when plaques became visible, the monolayers were stained with X-gal, and blue plaques or individual blue cells were counted.
- Fig. 15: Initiation of replication of rdAd DNA using a mutant Ad5 pTP. Same as Fig. 4, however, the G's at positions one, four, and seven in the Ad5 ITR are replaced by C's, and the wild-type Ad5 pTP is replaced by a mutant variant capable of binding dGMP instead of dCMP, or, alternatively, by a hybrid pTP consisting of the precursor part of wild-type Ad5 pTP, and the TP part of CELO^{PHELPS}.
- Fig. 16: Hybrid pTP. Shown is a schematic representation of hpTP-G, carrying the precursor part of wild-type Ad5 pTP, and the TP part of CELO^{PHELPS}.
- Fig. 17: Three nucleotide and amino acid sequences for CELO^{PHELPS} precursor to the terminal protein or hybrids thereof
a) Avian Adenovirus CELO^{PHELPS} pTP.
b) Hybrid pTP (precursor part derived from Ad5 and TP part derived from CELO^{PHELPS}.
c) Hybrid pTP (containing pTP of CELO^{PHELPS} with the translation start of Ad5 pTP.)

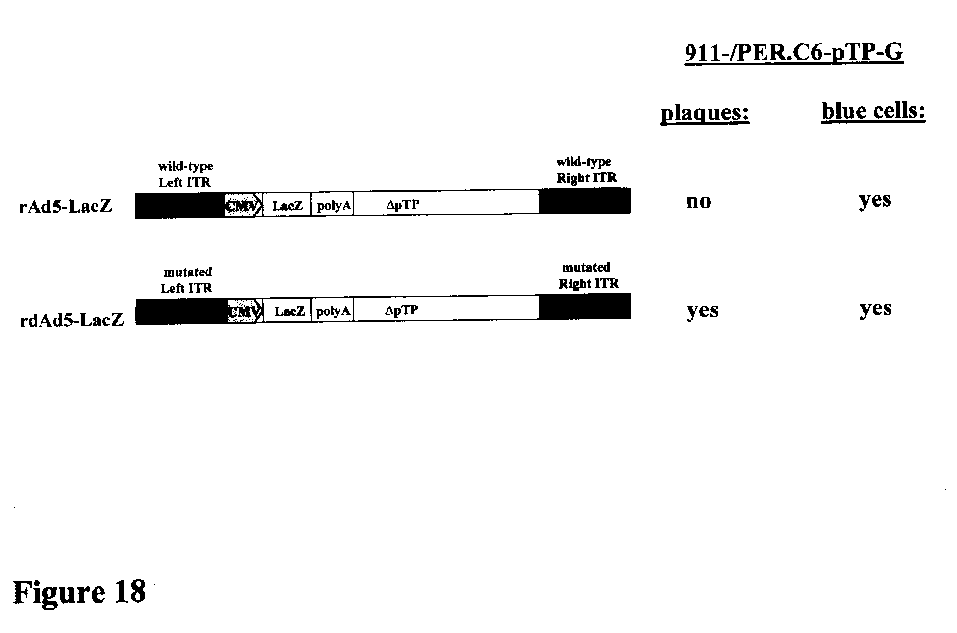

- Fig. 18: rdAd5-LacZ DNA replicates in 911-pTP-G or PER.C6-pTP-G cells. 10 µg rAd5-LacZ or rdAd5-LacZ DNA was transfected into sub-confluent 911-pTP-G or PER.C6-pTP-G cells in 6-well plates (two wells for each transfection). One well was overlayed with agar-containing culture medium, while the other well received normal liquid medium. After seven days, when plaques became visible, the monolayers were stained with X-gal, and blue plaques or individual blue cells were counted.
- Fig. 19: The use of a rdAd helper virus for gAd production.
- Fig. 20: Combination of rdAd technology with the cre-lox system for production of gAds.

### References

**Amalfitano, A., Hauser, M.A., Hu, H., Serra, D., Begy, C.R., and Chamberlain, J.S.** (1998): Production and characterization of improved adenovirus vectors with the E1, E2b, and E3 genes deleted. J. Virol. 72, 926-933.
**Alkhatib, G., and Briedis, D.J.** (1988): High-level eukaryotic *in vivo* expression of biologically active measles virus hemagglutinin by using an adenovirus type 5 helper-free vector system. J. Virol. 62, 2718-2727 .
**Andersson, M., Paabo, S., Nilsson, T., and Peterson, P.A.** (1985): Cell 43, 215-222.
**Bergelson, J.M., Cunningham, J.A., Kurt-Jones, E.A., Krithivas, A., Hong, J.S., Horwitz, M.S., Crowell, R.L., and Finberg, R.W.** (1997): Isolation of a common receptor for Coxsackie B viruses and adenoviruses 2 and 5. Science 275(5304), 1320-1323.
**Berk, A. J.** (1986): Adenovirus promoters and E1A transactivation. Ann. Rev. Genet. 20, 45-79.
**Bett, A. J., Prevec, L., and Graham, F. L.** (1993): Packaging Capacity and Stability of Human Adenovirus Type-5 Vectors. J Virol 67, 5911-5921.
**Botting, C.H., and Hay, R.T.** (1999): Characterization of the adenovirus preterminal protein and its interaction with the POU homeodomain of NFIII (Oct-1). Nucleic Acids Res. 27(13), 2799-2805.
**Bout, A., Imler, J. L., Schulz, H., Perricaudet, M., Zurcher, C., Herbrink, P., Valerio, D., and Pavirani, A.** (1994a): In vivo adenovirus-mediated transfer of human CFTR cDNA to Rhesus monkey airway epithelium: efficacy, toxicity and safety. Gene Therapy 1, 385-394.
**Bout, A., Perricaudet, M., Baskin, G., Imler, J. L., Scholte, B. J., Pavirani, A., and Valerio, D.** (1994b): Lung gene therapy: in vivo adenovirus mediated gene transfer to rhesus monkey airway epithelium. Human Gene Therapy 5, 3-10.
**Burgert, H.-G. and Kvist, S.** (1985): An adenovirus type 2 glycoprotein blocks cell surface expression of human histocompatibility class I antigens. Cell 41, 987-997.
**Challberg, J.W. and Rawlins, D.R.** (1984): Template requiremenrs for the initiation of adenovirus DNA replication. Proc. Natl. Acad. Sci. USA 81, 100-104. **Chiocca, S., Kurzbauer, R., Schaffner, G., Baker, A., Mautner, V., and Cotton, M.** (1996): The complete DNA sequence and genomic organization of the avian adenovirus CELO. J. Virol. 70(5), 2939-2949.
**Chroboczek, J. Bieber, F., and Jacrot, B.** (1992): The sequence of the genome of adenovirus type 5 and its comparison with the genome of adenovirus type 2. Virology 186, 280-285.
**Crystal, R.G., McElvaney, N.G., Rosenfeld, M.A., Chu, C.S., Mastrangeli, A., Hay, J.G., Brody, S.L., Jaffe, H.A., Eissa, N.T., and Danel, C.** (1994): Administration of an adenovirus containing the human CFTR cDNA to the respiratory tract of individuals with cystic fibrosis. Nature Genet. 8, 42-51. **Eloit, M., Gilardi-Hebenstreit, P., Toma, B., and**
**Perricaudet, M.** (1990): Construction of a defective adenovirus vector expressing the pseudorabies virus glycoprotein gp50 and its use as a live vaccine. J. Gen. Virol. 71, 2425- .
**Engelhardt, J. F., Litzky, L., and Wilson, J. M.** (1994a): Prolonged transgene expression in cotton rat lung with recombinant adenoviruses defective in E2A. Hum. Gene Ther. 5, 1217-1229.
**Engelhardt, J. F., Simon, R. H., Yang, Y., Zepeda, M., Weber-Pendleton, S., Doranz, B., Grossman, M., and Wilson, J. M.** (1993) : Adenovirus-mediated transfer of the CFTR gene to lung of nonhuman primates: biological efficacy study. Human Gene Therapy 4, 759-769.
**Engelhardt, J. F., Ye, X., Doranz, B., and Wilson, J. M.** (1994b): Ablation of E2A in recombinant adenoviruses improves transgene persistence and decreases inflammatory response in mouse liver. Proc Natl Acad Sci U S A 91, 6196-6200. **Ensinger, M.G. and Ginsberg, H.S.** (1972): Selection and preliminary characterization of temperature sensitive mutants of type 5 adenovirus. J. Virol. 10, 328-339.
**Fallaux, F.J., Bout, A.,Van der Velde, I., Van den Wollenberg, D.J.M., Hehir, K.M., Keegan, J., Auger, C., Cramer, S.J.,Van Ormondt, H.,Van der Eb, A.J., Valerio, D., and Hoeben, R.C.** (1998): New helper cells and matched early region 1-deleted adenovirus vectors prevent generation of replication-competent adenoviruses. Hum. gene Ther. 9, 1909-1917.
**Fallaux, F.J., Kranenburg, O., Cramer, S.J., Houweling, A., Van Ormondt, H., Hoeben, R.C., and Van der Eb, A.J.** (1996): Characterization of 911: A new helper cell line for the titration and propagation of early region 1-deleted adenoviral vectors. Hum. gene Ther. 7, 215-222.
**Flint, S.J.** (1980). Transformation by adenoviruses. In Molecular biology of tumor viruses, II. DNA tumor viruses, 2nd ed. J.Tooze ,ed. (Cold Spring Harbor Laboratory Press, Cold Spring Harbor, N.Y.) pp. 547-576.
**Fredman, J.N., and Engler, J.A.** (1993): Adenovirus precursor to terminal protein interacts with the nuclear matrix in vivo and in vitro. J. Virol. 67, 3384-3395.
**Fredman, J.N., Pettit, S.C., Horwitz, M.S., and Engler, J.A.** (1991): Linker insertion mutants in the adenovirus preterminal protein that affect DNA replication activity in vivo and in vitro. J. Virol. 65, 4591-4597.
**Freimuth, P.I. and Ginsberg, H.S.** (1986): Codon insertion mutants of the adenovirus terminal protein. Proc. Natl. Acad. Sci. USA 83, 7816-7820.
**Gallimore, P.H., Grand, R.J.A., and Byrd, P.J.** (1987): Transformation of human embryo retinoblasts with simian virus 40, adenovirus and ras oncogenes. Anticancer Res. 6, 499-508.
**Gooding, L.R. and Wold, W.S.M.** (1990): Molecular mechanisms by which adenoviruses counteract antiviral immune defences. Critical reviews in Immunology 10, 53-71.
**Graham, F.L. and Prevec, L.** (1991). Manipulation of adenovirus vectors. In Methods in Molecular Biology. Vol.7: Gene transfer and Expression Protocols. E.J. Murray, ed. (The Humana Press, Clifton, NJ) pp. 109-128.
**Graham, F. L., Smiley, J., Russell, W. C., and Nairn, R.** (1977): Characteristics of a human cell line transformed by DNA from adenovirus type 5. J. Gen. Virol. 36, 59-72. **Harris, M.P., and Hay, R.T.** (1988): DNA sequences required for the initiation of adenovirus serotype 4 DNA replication in vitro. J. Mol. Biol. 201, 57-67.
**Hay, R.T.** (1985): Origin of adenovirus DNA replication. Role of nuclear factor I binding site in vivo. J. Mol. Biol. 186, 129-136.
**Hay, R.T., Freeman, A., Leith, I., Monaghan, A., and Webster, A.** (1995): Molecular interactions during adenovirus replication. Curr. Top. Microbiol. Immunol. 199, 31-48. **He, T.C., Zhou, S., da Costa, L.T., Yu, J., Kinzler, K.W., and Vogelstein, B.** (1998): A simplified system for generating recombinant adenoviruses. Proc. Natl. Acad. Sci. USA 95(5), 2509-2514.
**Hehir, K.M., Armentano, D., Cardoza, L.M., Choquette, T.L., Berthelette, P.B., White, G.A., Couture, L.A., Everton, M.B., Keegan, J., Martin, J.M., Pratt, D.A., Smith, M.P., Smith, A.E., and Wasworth, S.C.** (1996): Moleculer characterization of replication-competent variants of adenoviral vectors and genome modifications to prevent their occurrence. J. vir. 70, 8459-8467.
**Van den Heuvel, S.J.L., The, S.I., Klein, B., Jochemsen, A.G., Zantema, A., and van der Eb, A.J.** (1992). p53 shares an antigenic determinant with proteins of 92 and 150 kilodaltons that may be involved in senescence of human cells. J.Virol. 66, 591-595.
**Horwitz, M. S.** (1990): Adenoviridae and their replication, pp. 1679-1740. In B. N. Fields, and D. M. Knipe (Eds): Virology, Raven Press, Ltd, New York.
**Hsu, K.H.L., Lubeck, M.D., Bhat, B.M., etc.** (1994): Efficacy of adenovirus-vectored respiratory syncytial virus vaccines in a new ferret model. Vaccine 12, 607-612 .
**Imler, J.-L.** (1995): Adenovirus vectors as recombinant viral vaccines. Vaccine 13(13), 1143-1151.
**Imler, J.-L., Bout, A., Dreyer, D., Dieterle, A., Schultz, H., Valerio, D., Mehtali, M., and Pavirani, A.** (1995): *Trans*-complementation of El-deleted adenovirus: a new vector to reduce the possibility of co-dissemination of wild-type and recombinant adenoviruses. Hum Gene Ther. 6, 711-721. **King, A.J. and Van der Vliet, P.C.** (1994): A precursor terminal protein-trinucleotide intermediate during initiation of adenovirus DNA replication: regeneration of molecular ends in vitro by a jumping back mechanism. EMBO J. 13(23), 5786-5792.
**Kruijer, W., Nicolas, J. C., Van Schaik, F. M. A., and Sussenbach, J. S.** (1983): Structure and function of DNA binding proteins from revertants of adenovirus type 5 mutants with a temperature-sensitive DNA replication. Virology 124, 425-433.
**Kruijer, W., Van Schaik, F. M. A., and Sussenbach, J. S.** (1981): Structure and organization of the gene coding for the DNA binding protein of adenovirus type 5. Nucleic Acids Res. 9, 4439-4457.
**Lally, C., Dorper, T., Gröger, W., Antoine, G., and Winnacker, E-L.** (1984): A size analysis of the adenovirus replicon. EMBO J. 3, 333-337.
**Leppard, K.N.** (1997): E4 gene function in adenovirus, adenovirus vector, and adeno-associated virus infections. J. gen. Virol. 78, 2131-2138.
**Levrero, M., Barban, V., Manteca, S., Balley, A., Balsamo, C., Avantaggiati, M.L., Natoli, G., Skellekens, H., Tiollais,**
**P., and Perricaudet, M.** (1991): Defective and nondefective adenovirus vectors for expressing foreign genes *in vitro* and *in vivo.* Gene 101, 195- 202.
**Lochmuller, H., Jani, A., Huard, J., Prescott, S., Simoneau, M., Massie, B., Karpati, G.,** and Acsadi, G. (1994): Emergence of early region 1-containing replication-competent adenovirus in stocks of replication-defective adenovirus recombinants (A E1 + ΔE3) during multiple passages in 293 cells. Hum. Gene Ther. 5, 1485-1492.
**Mitani, K., Graham, F.L., Caskey, C.T., and Kochanek, S.** (1995): Rescue, propagation, and partial purification of a helper virus-dependent adenovirus vector. Proc. Natl. Acad. Sci. USA 92, 3854-3858.
**Morsy, M.A. and Caskey, C.T.** (1999): Expanded-capacity adenoviral vectors-the helper-depended vectors. Mol medicine today, 5(1), 18-24.
**Mueller, R.E., Muldoon, R.L., and Jackson, G.G.** (1969): Communicability of enteric live adenovirus type 4 vaccine in families. J. Infect. Dis. 119, 60-66.
**Nagata, K., Guggenheimer, R.A., and Hurwitz, J.** (1983): Adenovirus DNA replication in vitro: Synthesis of full-lenght DNA with purified proteins. Proc. Natl Acad. Sci. USA 60, 4266-4270.
**Natuk, R.J., Lubeck, M.D., Chanda, P.K., Chengalvala M, Wade MS, Murthy SC, Wilhelm J, Vernon SK, Dheer SK, Mizutani S, *et al.*** (1993) : Immunogenicity of recombinant adenovirus-human immunodeficiency virus vaccines in chimpanzees. AIDS Res. Hum. Retr. 9, 395-404.
**Nevins, J.R., Ginsberg, H.S., Blanchard, J.M., Wilson, M.C., and Darnell, J.E.** (1979): Regulation of the primary expression of the early adenovirus transcription units. J. Virol. 32, 727-733.
**Nicolas, J. C., Suarez, F., Levine, A. J., and Girard, M.** (1981): Temperature-independent revertants of adenovirus H5ts125 and H5ts107 mutants in the DNA binding protein: isolation of a new class of host range temperature conditional revertants. Virology 108, 521-524.
**Van Ormondt, H., and Galibert, F.** (1984): Nucleotide sequences of adenovirus DNAs, pp. 73-142. In W. Doerfler (Ed): Current Topics in Microbiology and Immunology: The Molecular Biology of Adenoviruses 2, Springer-Verlag, Berlin Heidelberg New York Tokyo.
**Ostrove, J. M.** (1994): Safety testing programs for gene therapy viral vectors. Cancer Gene Ther. 1, 125-131. **Pacini, D.L., Dubovi, E.J., and Clyde, W.A.** (1984): A new animal model for human respiratory tract disease due to adenovirus. J. Infect. Dis. 150, 92-97.
**Parks, R.J., Chen, L., Anton, M., Sankar, U., Rudnicki, M.A., Graham, F.L.** (1996): A helper-dependent adenovirus vector system: removal of helper virus by Cre-mediated excision of the viral packaging signal. Proc. Natl. Acad. Sci. U S A 93(24):13565-13570.
**Pettit, S.C., Horwitz, M.S., and Engler, J.A.** (1989): Mutatations of the precursor to the terminal proteins of adenovirus serotypes 2 and 5. J. Virol. 63, 5244-5250. **Phelps, W.C., Yee, C.L., Munger, K., and Howley, P.M.** (1988): The human pappilloma virus type 16 E7 gene encodes transactivation and transformation functions similar to those of adenovirus E1A. Cell 53, 539-547.
**Prevec, L., Schneider, M., Rosenthal, K.L., Belbeck, L.W., Derbyshire, J.B., and Graham, F.L.** (1989): Use of human adenovirus-based vectors for antigen expression in animals. J. Gen. Virol. 70, 429-434.
**Prince, G.A., Porter, D.D., Jenson, A.B., Horswood, R.L., Chanock R.M., and Ginsberg, H.S.** (1993): Pathogenesis of adenovirus type 5 pneumonia in cotton rats *(Sigmodon hispidus).* J. Virol. 67, 101-111.
**Pruijn, G.J.M., Van Driel, W., and Van der Vliet, P.C.** (1986) : Nuclear Factor III, a novel sequencespecific DNAbinding protein from HeLa cells stimulating Adenovirus DNA replication. Nature 322, 656-659.
**Ragot, T., Finerty, S., Watkins, P.E., Perricaudet, M., and Morgan, A.J.** (1993): Replication-defective recombinant adenovirus expressing the Epstein-Barr virus (EBV) envelope glycoprotein gp340/220 induces protective immunity against EBV-induced lymphomas in the cottontop tamarin. J. Gen. Virol 74, 501-507.
**Roovers, D.J., Overman, P.F/, Chen, C.Q., and Sussenbach, J.S.** (1991): Linker mutation scanning of the genes encoding the adenovirus type 5 terminal protein precursor and DNA polymerase. Virology 180, 273-284.
**Rosenfeld, P.J., O'Neill, E.A., Wides, R.J., and Kelly, T.J.** (1987): Sequence-specific interactions between cellular DNA-binding proteins and the adenovirus origin of DNA replication. Mol. Cell Biol. 7, 875-886.
**Schwartz, A.R., Togo, Y., and Hornick, R.B.** (1974): Clinical evaluation of live types 1, 2, and 5 adenovirus vaccines. Am. Rev. Resp. Dis. 109, 233-239.
**Signas, C., Katze, M.G., Persson, H., and Philipson, L.** (1982) : An adenovirus glycoprotein binds heavy chains of class I transplantation antigens from man and mouse. Nature 299, 175-178.
**Simon, R. H., Engelhardt, J. F., Yang, Y., Zepeda, M., Weber-Pendleton, S., Grossman, M., and Wilson, J. M.** (1993): Adenovirus-mediated transfer of the CFTR gene to lung of nonhuman primates: toxicity study. Human Gene Therapy 4, 771-780.
**Stratford-Perricaudet, L. D., and Perricaudet, M.** (1991): Gene transfer into animals: the promise of adenovirus, pp. 51-61. In O. Cohen-Adenauer, and M. Boiron (Eds): Human Gene Transfer, John Libbey Eurotext.
**Tevethia, M.J., Spector, D.J., Leisure, K.M., and Stinsky, M.F.** (1987): Participation of two human cytomegalovirus early gene regions in transcriptional activation of adenovirus promoters. Virology 161, 276-285.
**Tooze, J.** (1981): DNA Tumor Viruses (revised). Cold Spring Harbor Laboratory. Cold Spring Harbor, New York.
**Top, F.H., Buesher, E.L., Bancroft, W.H., and Russel, P.K.** (1971a): Immunization with live type 7 and 4 vaccines. II. Antibody response and protective effect against accute respiratory disease due to adenovirus type 7. J. Infect. Dis. 124, 155-160.
**Top, F.H., Grossman, R.A., Bartelloni, P.J. , Segal HE, Dudding BA, Russell PK, Buescher EL** (1971b): Immunization with live type 7 and 4 vaccines. I. Safety, infectivity, antigenicity, and potency of adenovirus type 7 vaccine in humans. J. Infect. Dis. 124, 148-154.
**Vaessen, R.T.M.J., Houweling, A, Israel, A., Kourilsky, P., and van der Eb, AJ.** (1986). Adenovirus ElA-mediated regulation of class I MHC expression. EMBO J. 5, 335-341. **Vales, L.D. and Darnell, J.E.** (1989): Promoter occlusion prevents transcription of adenovirus polypeptide IX mRNA until after DNA replication. Genes Dev. 3(1), 49-59. **Van der Vliet, P.C.** (1995): Adenovirus DNA replication. Curr. Top. Microbiol. Immunol. 199, 1-30.
**Webster, A., Leith, I., and Hay, R.T.** (1997): Domain organization of the preterminal protein. J. Virol. 71, 539-547.
**Whittaker, J.L., Byrd, P.J., Grand, R.J.A., and Gallimore, P.H.** (1984). Isolation and characterization of four adenovirus type 12-transformed human embryo kidney cell lines. Mol.Cell Biol. 4, 110-116.
**Wides, R.J., Challberg, M.D., Rawlins, D.R., and Kelly, T.J.** (1987): Adenovirus origins of DNA replication: Sequence requirements for replication in vitro. Mol. Cell Biol. 7, 864-874.
**Yang, Y., Li, Q., Ertl, H. C. J., and Wilson, J. M.** (1995): Cellular and humoral immune responses to viral antigens create barriers to lung-directed gene therapy with recombinant adenoviruses. J. Virol. 69, 2004-2015.
**Yang, Y., Nunes, F. A., Berencsi, K., Furth, E. E., Gonczol, E., and Wilson, J. M.** (1994a): Cellular immunity to viral antigens limits El-deleted adenoviruses for gene therapy. Proc Natl Acad Sci U S A 91, 4407-11.
**Yang, Y., Nunes, F. A., Berencsi, K., Gonczol, E., Engelhardt, J. F., and Wilson, J. M.** (1994b): Inactivation of E2A in recombinant adenoviruses improves the prospect for gene therapy in cystic fibrosis. Nat Genet 7, 362-369. **Yeh, P., Dedieu, J.FF., Orsini, C., Vigne, E., Denefle, P., and Perricaudet, M.** (1996): Efficient dual transcomplementation of adenovirus E1 and E4 regions from a 293-derived cell line expressing a minimal E4 functional unit. J. Vir. 70, 559-565.
**Zabner, J., Couture, L.A., Gregory, R.J., Graham, S.M., Smith, A.E., and Welsh, M.J.** (1993): Adenovirus-mediated gene transfer transiently corrects the chloride transport defect in-nasal epithelia of patients with cystic fibrosis. Cell 75, 207-216.
**Patent application number E** 95201611.1, 15 June 1995

## Claims

1. A chimaeric adenoviral vector comprising a functional packaging signal from an adenovirus and comprising a site for protein primed replication initiation not present in the wild type adenovirus from which said packaging signal originates.

2. A chimaeric adenoviral vector according to claim 1, wherein said site for protein primed replication initiation is derived from a phage and/or a virus comprising an essentially linear DNA genome.

3. A chimaeric adenoviral vector according to claim 2, wherein said virus is an adenovirus.

4. A chimaeric adenoviral vector according to anyone of claims 1-3, wherein said site for protein primed replication initiation comprises a cytosine residue in the bottom strand, at at least two of the positions 1, 4, 7 and onward at intervals of three nucleotides.

5. A chimaeric adenoviral vector according to anyone of claims 1-4, wherein said site for protein primed replication initiation comprises a cytosine residue in the bottom strand, at at least two of the positions 1, 4 and 7.

6. A chimaeric adenoviral vector according to anyone of claims 1-5, wherein the adenovirus from which said packaging signal is derived comprises a site for protein primed replication initiation comprising a guanine residue in the bottom strand, at at least two of the positions 1, 4, 7 and onward at intervals of three nucleotides.

7. A chimaeric adenoviral vector according to claim 6, wherein said site for protein primed replication initiation comprises a guanine residue in the bottom strand, at at least two of the positions 1, 4 and 7.

8. A kit of parts capable of starting replication of a chimaeric adenoviral vector according to any one of claims 1-7, comprising a functional precursor terminal protein capable of binding to a site for protein primed replication initiation on said adenoviral vector and an adenovirus polymerase capable of binding to said precursor terminal protein.

9. A kit of parts according to claim 8, wherein at least a nucleotide binding part of said precursor terminal protein does not originate from the wild type adenovirus from which said packaging signal originates.

10. A kit of parts according to claim 8 or claim 9, wherein said precursor terminal protein is also capable of binding to Oct-1.

11. A kit of parts according to any one of claims 8-10, wherein said polymerase is also capable of binding to NFI.

12. A kit of parts according to any one of claims 8-11, wherein said polymerase is a fusion protein comprising polymerase activity from a first adenovirus and comprising precursor terminal protein binding activity from a second adenovirus, wherein said first adenovirus comprises a site for protein primed replication initiation not present in said second adenovirus.

13. A kit of parts according to any one of claims 8-12, wherein said precursor terminal protein is a fusion protein comprising polymerase binding activity from a first adenovirus and comprising binding activity for a site for protein primed replication initiation from a second adenovirus, wherein said first adenovirus comprises a site for protein primed replication initiation not present in said second adenovirus.

14. A method for producing a rescue defective adenoviral gene delivery vehicle comprising contacting a chimaeric adenoviral vector according to anyone of claims 1-7 with a kit of parts according to anyone of claims 8-13 under conditions allowing for replication and packaging of said adenoviral gene delivery vehicle.

15. A method for providing at least a partial helper function for the production of adeno-associated virus and/or adeno-associated virus vectors in a cell comprising providing said cell with a chimaeric adenoviral vector according to anyone of claims 1-7.

16. A method for the production of a minimal adenovirus vector without essentially also producing other adenovirus vectors comprising providing a cell with said minimal adenovirus vector and an adenovirus vector according to anyone of claims 1-7, wherein said minimal adenovirus vector comprises an essentially different site for protein primed replication initiation than the site for protein primed replication initiation in said adenovirus vector.


### Annex to the apllication documents - subsequently filed sequences listing

SEQUENCE LISTING

<110> Leids Universitair Medisch Centrum

<120> Conditional Replication of Recombinant Human Adenovirus
      DNA Carrying Modified Inverted Terminal Repeat
      sequences

<130> P50533EP00

<140> 99204064.2
<141> 1999-12-01

<160> 55

<170> PatentIn Ver. 2.1

<210> 1
<211> 8
<212> DNA
<213> adenoviridae

<220>
<221> misc_feature
<222> (1)..(8)
<223> /note="Replication sequence"

<400> 1
gtagtagt                                                          8


<210> 2
<211> 8
<212> DNA
<213> adenoviridae

<220>
<221> misc_feature
<222> (1)..(8)
<223> /note="Replication sequence"

<400> 2
ctactact                                                          8


<210> 3
<211> 11
<212> DNA
<213> adenoviridae

<220>
<221> misc_feature
<222> (1)..(11)
<223> /note="Replication sequence"

<400> 3
ctactactac t                                                      11


<210> 4
<211> 45
<212> DNA
<213> adenoviridae

<220>
<221> primer_bind

<222> (1)..(45)

<400> 4
cgaatttttt cttaattaag atgatgtata atatacctta ttttg                    45


<210> 5
<211> 31
<212> DNA
<213> adenoviridae

<220>
<221> primer_bind
<222> (1)..(31)

<400> 5
cggtaccatc gatagtatta cgcgctatga g                                  31


<210> 6
<211> 23
<212> DNA
<213> adenoviridae

<220>
<221> primer_bind
<222> (1)..(23)

<400> 6
ccctaggcaa aatagcaccc tcc                                           23


<210> 7
<211> 39
<212> DNA
<213> adenoviridae

<220>
<221> primer_bind
<222> (1)..(39)

<400> 7
tggatcccga tcgttaatta agatgatgta taatatacc                          39


<210> 8
<211> 34
<212> DNA
<213> adenoviridae

<220>
<221> primer_bind
<222> (1)..(34)

<400> 8
taaagcttgc caccatggcc ttgagcgtca acga                               34


<210> 9
<211> 28
<212> DNA
<213> adenoviridae

<220>
<221> primer_bind
<222> (1)..(28)

<400> 9
gactgcagag ttggaagacg ccgcccgt                                        28


<210> 10
<211> 29
<212> DNA
<213> adenoviridae

<220>
<221> primer_bind
<222> (1)..(29)

<400> 10
ttggatcctt acagaggctg acctcgtcg                                       29


<210> 11
<211> 106
<212> DNA
<213> adenoviridae

<220>
<221> misc_feature
<222> (1)..(106)
<223> /note="Part of the ITR/origin of replication"

<400> 11
catcatcaat aatatacctt attttggatt gaagccaata tgataatgag ggggtagtag 60
ttattatatg gaataaaacc taacttcggt tatactatta ctcccc                    106


<210> 12
<211> 51
<212> DNA
<213> adenoviridae

<220>
<221> misc_feature
<222> (1)..(51)
<223> /note="AAd"

<400> 12
cntcatctat aataacctca aaaactaacg cagtcataac cgaccataac c              51


<210> 13
<211> 51
<212> DNA
<213> adenoviridae

<220>
<221> misc_feature
<222> (1)..(51)
<223> /note="PAd2"

<400> 13
catcatcaat aatataccgc acacttttat tgcccctagg gttgtgttct g              51


<210> 14
<211> 51
<212> DNA
<213> adenoviridae

<220>
<221> misc_feature
<222> (1)..(51)
<223> /note="PAd3"

<400> 14
catcatcaat aatataccgc acacttttat tgcccctttt gtggcgtggt g          51


<210> 15
<211> 48
<212> DNA
<213> adenoviridae

<220>
<221> misc_feature
<222> (1)..(48)
<223> /note="PAd5"

<400> 15
catcatcaat aatatacgga acaaaaatgc gtgatgacgt agacggcg          48


<210> 16
<211> 49
<212> DNA
<213> adenoviridae

<220>
<221> misc_feature
<222> (1)..(49)
<223> /note="BAd2"

<400> 16
catcatcaat aatatacggt gcattttgtg cgtgatgacg tatacagcg          49


<210> 17
<211> 50
<212> DNA
<213> adenoviridae

<220>
<221> misc_feature
<222> (1)..(50)
<223> /note="BAd"

<400> 17
catcatcaat aatatacgga acacttttgc gtgatgacat tgacgtgctg          50


<210> 18
<211> 51
<212> DNA
<213> adenoviridae

<220>
<221> misc_feature
<222> (1)..(51)
<223> /note="TAd"

<400> 18
catcatcaat aatatacctg acacttttga cgtaatgacg tcagacgtaa g          51


<210> 19

<211> 49
<212> DNA
<213> adenoviridae

<220>
<221> misc_feature
<222> (1)..(49)
<223> /note="CAd1"

<400> 19
catcatcaat aatatacagg acaaagaggt gtggcctaaa tgttgtttt          49


<210> 20
<211> 49
<212> DNA
<213> adenoviridae

<220>
<221> misc_feature
<222> (1)..(49)
<223> /note="CAd"

<400> 20
catcatcaat aatatacagg acaaagaggt gtggcctaaa tgttgtttt          49


<210> 21
<211> 49
<212> DNA
<213> adenoviridae

<220>
<221> misc_feature
<222> (1)..(49)
<223> /note="CAd2"

<400> 21
catcatcaat aatatacagg acaaagaggt gtggcttaaa tttgggtgt          49


<210> 22
<211> 50
<212> DNA
<213> adenoviridae

<220>
<221> misc_feature
<222> (1)..(50)
<223> /note="MAd1"

<400> 22
catcatcaat aatatacagt tagcaaaaaa tggcgccttt gtttggcttt          50


<210> 23
<211> 51
<212> DNA
<213> adenoviridae

<220>
<221> misc_feature
<222> (1)..(51)
<223> /note="BAd3"

<400> 23

catcatcaat aatctacagt acactgatgg cagcggtcca actgccaatc a 51

<210> 24
<211> 50
<212> DNA
<213> adenoviridae

<220>
<221> misc_feature
<222> (1)..(50)
<223> /note="EAd"

<400> 24
catcatcaat aatatacagg acacacgggc atggggccaa gaaaggggag 50

<210> 25
<211> 51
<212> DNA
<213> adenoviridae

<220>
<221> misc_feature
<222> (1)..(51)
<223> /note="HAd10"

<400> 25
cttcatcaat aatatacccc acaaagtaaa caaaagttaa tatgcaaatg a 51

<210> 26
<211> 51
<212> DNA
<213> adenoviridae

<220>
<221> misc_feature
<222> (1)..(51)
<223> /note="HAd9"

<400> 26
ctatctatat aatatacccc acaaagtaaa caaaagttaa tatgcaaatg a 51

<210> 27
<211> 51
<212> DNA
<213> adenoviridae

<220>
<221> misc_feature
<222> (1)..(51)
<223> /note="HAd4"

<400> 27
ctctctctat aatatacctt attttttttg tgtgagttaa tatgcaaata a 51

<210> 28
<211> 50
<212> DNA
<213> adenoviridae

<220>
<221> misc_feature

<222> (1)..(50)
<223> /note="HAd7"

<400> 28
ctatctatat aatatacctt atagatggaa tggtgccaat atgtaaatga          50


<210> 29
<211> 38
<212> DNA
<213> adenoviridae

<220>
<221> misc_feature
<222> (1)..(38)
<223> /note="HAd3"

<400> 29
agatacctta agatggaatg gtgccaacat gtaaatga                      38


<210> 30
<211> 50
<212> DNA
<213> adenoviridae

<220>
<221> misc_feature
<222> (1)..(50)
<223> /note="HAd34"

<400> 30
catcatcaat aatatacctt atagatggaa tggtgccaat atgtaaatga          50


<210> 31
<211> 50
<212> DNA
<213> adenoviridae

<220>
<221> misc_feature
<222> (1)..(50)
<223> /note="HAd40"

<400> 31
catcatcaat aatatacctt aaaactggaa acgagccaat atgataatga          50


<210> 32
<211> 50
<212> DNA
<213> adenoviridae

<220>
<221> misc_feature
<222> (1)..(50)
<223> /note="HAd41"

<400> 32
catcatcaat aatatacctt aaagctggaa acgagccaat atgataatga          50


<210> 33
<211> 49
<212> DNA

<213> adenoviridae

<220>
<221> misc_feature
<222> (1)..(49)
<223> /note="HAd2"

<400> 33
catcatcaat aatatacctt attttggatt gaagccaata tgataatga          49


<210> 34
<211> 49
<212> DNA
<213> adenoviridae

<220>
<221> misc_feature
<222> (1)..(49)
<223> /note="HAd5"

<400> 34
catcatcaat aatatacctt attttggatt gaagccaata tgataatga          49


<210> 35
<211> 49
<212> DNA
<213> adenoviridae

<220>
<221> misc_feature
<222> (1)..(49)
<223> /note="SAd30"

<400> 35
catcatcaat aatatacctt atttgggaac ggtgccaata tgctaatga          49


<210> 36
<211> 49
<212> DNA
<213> adenoviridae

<220>
<221> misc_feature
<222> (1)..(49)
<223> /note="HAd12"

<400> 36
catcatcaat aatatacctt atactggact agtgccaata ttaaaatga          49


<210> 37
<211> 49
<212> DNA
<213> adenoviridae

<220>
<221> misc_feature
<222> (1)..(49)
<223> /note="HAd18"

<400> 37
catcatcaat aatatacctt atactggact agagccaata ttaaaatga          49

<210> 38
<211> 49
<212> DNA
<213> adenoviridae

<220>
<221> misc_feature
<222> (1)..(49)
<223> /note="HAd31"

<400> 38
catcatcaat aatatacctt acactggact tgagccaata ttaaaatga          49


<210> 39
<211> 4
<212> DNA
<213> adenoviridae

<220>
<221> misc_feature
<222> (1)..(4)
<223> /note = "various recognition sites"
<400> 39
acct                                                           4


<210> 40
<211> 5
<212> DNA
<213> adenoviridae

<220>
<221> misc_feature
<222> (1)..(5)
<223> /note = "various recognition sites"
<400> 40
cggaa                                                          5


<210> 41
<211> 5
<212> DNA
<213> adenoviridae

<220>
<221> misc_feature
<222> (1)..(5)
<223> /note = "various recognition sites"
<400> 41
tacgt                                                          5


<210> 42
<211> 4
<212> DNA
<213> adenoviridae

<220>
<221> misc_feature
<222> (1)..(4)
<223> /note = "various recognition sites"

<400> 42
atta                                                           4

<210> 43
<211> 10
<212> DNA
<213> adenoviridae

<220>
<221> misc_feature
<222> (1)..(10)
<223> /note = "various recognition sites"

<400> 43
ccatataata                                                              10


<210> 44
<211> 8
<212> DNA
<213> adenoviridae

<220>
<221> misc_feature
<222> (1)..(8)
<223> /note = "various recognition sites"

<400> 44
tgatgatg                                                                8


<210> 45
<211> 30
<212> DNA
<213> adenoviridae

<220>
<221> misc_feature
<222> (1)..(30)
<223> /note="Wild-type Ad5"

<400> 45
aatccaaaat aaggtatatt attgatgatg                                        30


<210> 46
<211> 30
<212> DNA
<213> adenoviridae

<220>
<221> misc_feature
<222> (1)..(30)
<223> note="CELOphelps"

<400> 46
cgttagtttt tgaggttatt atacatcatc                                        30


<210> 47
<211> 30
<212> DNA
<213> adenoviridae

<220>
<221> misc_feature
<222> (1)..(30)

<223> /note="CELOphelps/Ad5"

<400> 47
atccaaaata aggtatatta ttacatcatc                                        30


<210> 48
<211> 30
<212> DNA
<213> adenoviridae

<220>
<221> misc_feature
<222> (1)..(30)
<223> /note="Ad5/CELOphelps"

<400> 48
cgttagtttt tgaggttatt attgatgatg                                        30


<210> 49
<211> 49
<212> DNA
<213> adenoviridae

<220>
<221> misc_feature
<222> (1)..(49)
<223> /note="CELOphelps"

<400> 49
gatgatgtat aataacctca aaaactaacg cagtcataac cggccataa                   49


<210> 50
<211> 1944
<212> DNA
<213> adenoviridae

<220>
<223> Description of Combined DNA/RNA Molecule: Avian
      Adenovirus Celo Phelps pTP

<220>
<221> CDS
<222> (1)..(1941)

<400> 50
atg gcg ggg acg ggg tgc att acg agc ggt cta aaa acg tca ggg gct    48
Met Ala Gly Thr Gly Cys Ile Thr Ser Gly Leu Lys Thr Ser Gly Ala
  1               5                  10                  15

caa ttt cag gct tac aga gtc ttc gcg gat ctc acc gga cag agt cta    96
Gln Phe Gln Ala Tyr Arg Val Phe Ala Asp Leu Thr Gly Gln Ser Leu
             20                  25                  30

gcc acc gtc cgc ttc tgc gaa ccc gtc atc acc gct gac tat gtc ctc    144
Ala Thr Val Arg Phe Cys Glu Pro Val Ile Thr Ala Asp Tyr Val Leu
         35                  40                  45

gtt cgg gaa gaa cca gcc ctg gtc cac ccc cga agc gtg ttt ctc gtc    192
Val Arg Glu Glu Pro Ala Leu Val His Pro Arg Ser Val Phe Leu Val
     50                  55                  60

agc cgg gtg tac gac tac cgt tta atg caa ctc cga gac ctc gcg ccg    240
Ser Arg Val Tyr Asp Tyr Arg Leu Met Gln Leu Arg Asp Leu Ala Pro

65                  70                  75                  80

cga tcg ccg aac gtc gcc gcg ccg ccc tac aac gga ttg ccg ccg ccg     288
Arg Ser Pro Asn Val Ala Ala Pro Pro Tyr Asn Gly Leu Pro Pro Pro
                85                  90                  95

cac ctt ctc ctc ggg tac caa gct atg cac cgc gcg ctc aac gat tac     336
His Leu Leu Leu Gly Tyr Gln Ala Met His Arg Ala Leu Asn Asp Tyr
            100                 105                 110

ctt ttc gac aac cgc gtt ttt atg cag ata ggt tac gat agc cca ccc     384
Leu Phe Asp Asn Arg Val Phe Met Gln Ile Gly Tyr Asp Ser Pro Pro
        115                 120                 125

caa aga ccc aga cgc ctc ttt tgg acc tgt ctg acc gac tgc tcc tac     432
Gln Arg Pro Arg Arg Leu Phe Trp Thr Cys Leu Thr Asp Cys Ser Tyr
    130                 135                 140

gcc gtc aat gta ggg cag tac atg cga ttt ctc gat ctc gac aac ttt     480
Ala Val Asn Val Gly Gln Tyr Met Arg Phe Leu Asp Leu Asp Asn Phe
145                 150                 155                 160

cac ggt acg ttc acg cag atg cac aac gcc gta ctc atg gac cgc gtg     528
His Gly Thr Phe Thr Gln Met His Asn Ala Val Leu Met Asp Arg Val
                165                 170                 175

gcc gcg gac atg ggc cgg gcg cat ctg cga ggt agg gga atc gac gta     576
Ala Ala Asp Met Gly Arg Ala His Leu Arg Gly Arg Gly Ile Asp Val
            180                 185                 190

ggc cgt cac gga caa gtg ttg ccg cag ctc gac gcc gaa cac cac agc     624
Gly Arg His Gly Gln Val Leu Pro Gln Leu Asp Ala Glu His His Ser
        195                 200                 205

cta ctg tcg ggc aac gga gcg ggt ggc ttg caa gaa ggc gtc ctc atg     672
Leu Leu Ser Gly Asn Gly Ala Gly Gly Leu Gln Glu Gly Val Leu Met
    210                 215                 220

cga acg gcc tct gcc gcc gac gcc gaa ctg ctc gcc gcc atc cgc caa     720
Arg Thr Ala Ser Ala Ala Asp Ala Glu Leu Leu Ala Ala Ile Arg Gln
225                 230                 235                 240

cta aga gtc gcc ctc tgc cac tat cta ttc tgc tac gca tat gat cta     768
Leu Arg Val Ala Leu Cys His Tyr Leu Phe Cys Tyr Ala Tyr Asp Leu
                245                 250                 255

ttt caa acg gaa gaa aga tat cgg ttc tta cct gga tcc gat gtg ttc     816
Phe Gln Thr Glu Glu Arg Tyr Arg Phe Leu Pro Gly Ser Asp Val Phe
            260                 265                 270

ctt gaa cca aac tgg ctc tcc tac ttc gcg gaa gcc ttc gcg gag cta     864
Leu Glu Pro Asn Trp Leu Ser Tyr Phe Ala Glu Ala Phe Ala Glu Leu
        275                 280                 285

gac acc cag caa ctg gtg cgg gat gcc gag cgc aag ttt cga gga aga     912
Asp Thr Gln Gln Leu Val Arg Asp Ala Glu Arg Lys Phe Arg Gly Arg
    290                 295                 300

cgg gac gta gag gaa cct acg gaa aca atg gcg aga tgt ttc atg agc     960
Arg Asp Val Glu Glu Pro Thr Glu Thr Met Ala Arg Cys Phe Met Ser
305                 310                 315                 320

act cta gcg agc gac gcc gtt tcc tta gca gga acg ggt ctg tca gga
1008
Thr Leu Ala Ser Asp Ala Val Ser Leu Ala Gly Thr Gly Leu Ser Gly
                325                 330                 335

ggc gcc atc acc ctc tgc agc cgg cgg gta acc gac cgc acc ggc ctg
1056
Gly Ala Ile Thr Leu Cys Ser Arg Arg Val Thr Asp Arg Thr Gly Leu
            340                 345                 350

cgc cct aga gac cgc cac ggc aga gcc atc acc gcg tcc gaa gcg cgc
1104
Arg Pro Arg Asp Arg His Gly Arg Ala Ile Thr Ala Ser Glu Ala Arg
        355                 360                 365

cgc att agg ccc cgt gcc gtg cgg gcc ttc gta gac cgc ctg ccc cgc
1152
Arg Ile Arg Pro Arg Ala Val Arg Ala Phe Val Asp Arg Leu Pro Arg
    370                 375                 380

gtc acg cgg cgg cga cgg aga ccc ccc tcc ccc gcg ccc cct ccc gaa
1200
Val Thr Arg Arg Arg Arg Arg Pro Pro Ser Pro Ala Pro Pro Pro Glu
385                 390                 395                 400

gaa ata gaa gaa gcc gcc atg gaa gta gaa gaa cca gaa gag gag gaa
1248
Glu Ile Glu Glu Ala Ala Met Glu Val Glu Glu Pro Glu Glu Glu Glu
                405                 410                 415

gaa gag ctg tta gac gag gtg att cgc aca gcg ctc gaa gcc atc ggg
1296
Glu Glu Leu Leu Asp Glu Val Ile Arg Thr Ala Leu Glu Ala Ile Gly
            420                 425                 430

gca ctg caa gac gag ctc agc ggg gcc gcc cgg aga cac gaa ctc ttc
1344
Ala Leu Gln Asp Glu Leu Ser Gly Ala Ala Arg Arg His Glu Leu Phe
        435                 440                 445

agg ttt gcc aac gac ttc tac cgc atg ctc ctg acc gcg cgc gac gcg
1392
Arg Phe Ala Asn Asp Phe Tyr Arg Met Leu Leu Thr Ala Arg Asp Ala
    450                 455                 460

gga ctc atg gga gag tcg ttc ctg cgc aag tgg gtg ctg tac ttc ttc
1440
Gly Leu Met Gly Glu Ser Phe Leu Arg Lys Trp Val Leu Tyr Phe Phe
465                 470                 475                 480

tta gcc gaa cat atc gcc tct aca ctc tac tac ctg tac agc cac ttc
1488
Leu Ala Glu His Ile Ala Ser Thr Leu Tyr Tyr Leu Tyr Ser His Phe
                485                 490                 495

atc gcc aac cgc gag ttc cgc cgg tac gtc gac gtc ctg acc tta caa
1536
Ile Ala Asn Arg Glu Phe Arg Arg Tyr Val Asp Val Leu Thr Leu Gln
            500                 505                 510

gta ctc gtc gtg gga tgg gac gtc aac gcg cag cag gtt ttt aaa cgc
1584
Val Leu Val Val Gly Trp Asp Val Asn Ala Gln Gln Val Phe Lys Arg
        515                 520                 525

ata tgg agc gag caa tcc aac ccc gcc acc ata ttc gaa acc ctg tgg
1632
Ile Trp Ser Glu Gln Ser Asn Pro Ala Thr Ile Phe Glu Thr Leu Trp
    530                 535                 540

gaa cgt ata tta cgc gat ttt ctt atg atg gtc gaa cgg acg ggt caa
1680
Glu Arg Ile Leu Arg Asp Phe Leu Met Met Val Glu Arg Thr Gly Gln
545                 550                 555                 560

ttc gaa ggc atg gac gat gcg gac caa caa ctg ttt ctc tct gat att
1728
Phe Glu Gly Met Asp Asp Ala Asp Gln Gln Leu Phe Leu Ser Asp Ile
                565                 570                 575

caa tac aga gac cgc tcc ggt gac atc gaa gaa gtg ctg aag cag ctc
1776
Gln Tyr Arg Asp Arg Ser Gly Asp Ile Glu Glu Val Leu Lys Gln Leu
            580                 585                 590

aac ctc agc gaa gag ctg atc gat agc atc gac atc agt ttc cgc atc
1824
Asn Leu Ser Glu Glu Leu Ile Asp Ser Ile Asp Ile Ser Phe Arg Ile
        595                 600                 605

aaa ttt aaa ggc atc gta gcc atc gct acc aac gag gag atc aaa gcc
1872
Lys Phe Lys Gly Ile Val Ala Ile Ala Thr Asn Glu Glu Ile Lys Ala
    610                 615                 620

aac ctc aga cgc gtg ctc cgc cac cgc cgc gaa gac atc gaa gcg gcg
1920
Asn Leu Arg Arg Val Leu Arg His Arg Arg Glu Asp Ile Glu Ala Ala
625                 630                 635                 640

gcg cga cga ggt cag cct ctg taa
1944
Ala Arg Arg Gly Gln Pro Leu
                645


<210> 51
<211> 647
<212> PRT
<213> adenoviridae
<223> Description of Combined DNA/RNA Molecule: Avian
      Adenovirus Celo Phelps pTP

<400> 51
Met Ala Gly Thr Gly Cys Ile Thr Ser Gly Leu Lys Thr Ser Gly Ala
  1               5                  10                  15

Gln Phe Gln Ala Tyr Arg Val Phe Ala Asp Leu Thr Gly Gln Ser Leu
             20                  25                  30

Ala Thr Val Arg Phe Cys Glu Pro Val Ile Thr Ala Asp Tyr Val Leu
         35                  40                  45

Val Arg Glu Glu Pro Ala Leu Val His Pro Arg Ser Val Phe Leu Val
     50                  55                  60

Ser Arg Val Tyr Asp Tyr Arg Leu Met Gln Leu Arg Asp Leu Ala Pro
 65                  70                  75                  80

Arg Ser Pro Asn Val Ala Ala Pro Pro Tyr Asn Gly Leu Pro Pro Pro
                 85                  90                  95

His Leu Leu Leu Gly Tyr Gln Ala Met His Arg Ala Leu Asn Asp Tyr
            100                 105                 110

Leu Phe Asp Asn Arg Val Phe Met Gln Ile Gly Tyr Asp Ser Pro Pro

115                 120                 125

Gln Arg Pro Arg Arg Leu Phe Trp Thr Cys Leu Thr Asp Cys Ser Tyr
    130                 135                 140

Ala Val Asn Val Gly Gln Tyr Met Arg Phe Leu Asp Leu Asp Asn Phe
145                 150                 155                 160

His Gly Thr Phe Thr Gln Met His Asn Ala Val Leu Met Asp Arg Val
                165                 170                 175

Ala Ala Asp Met Gly Arg Ala His Leu Arg Gly Arg Gly Ile Asp Val
            180                 185                 190

Gly Arg His Gly Gln Val Leu Pro Gln Leu Asp Ala Glu His His Ser
        195                 200                 205

Leu Leu Ser Gly Asn Gly Ala Gly Gly Leu Gln Glu Gly Val Leu Met
    210                 215                 220

Arg Thr Ala Ser Ala Ala Asp Ala Glu Leu Leu Ala Ala Ile Arg Gln
225                 230                 235                 240

Leu Arg Val Ala Leu Cys His Tyr Leu Phe Cys Tyr Ala Tyr Asp Leu
                245                 250                 255

Phe Gln Thr Glu Glu Arg Tyr Arg Phe Leu Pro Gly Ser Asp Val Phe
            260                 265                 270

Leu Glu Pro Asn Trp Leu Ser Tyr Phe Ala Glu Ala Phe Ala Glu Leu
        275                 280                 285

Asp Thr Gln Gln Leu Val Arg Asp Ala Glu Arg Lys Phe Arg Gly Arg
    290                 295                 300

Arg Asp Val Glu Glu Pro Thr Glu Thr Met Ala Arg Cys Phe Met Ser
305                 310                 315                 320

Thr Leu Ala Ser Asp Ala Val Ser Leu Ala Gly Thr Gly Leu Ser Gly
                325                 330                 335

Gly Ala Ile Thr Leu Cys Ser Arg Arg Val Thr Asp Arg Thr Gly Leu
            340                 345                 350

Arg Pro Arg Asp Arg His Gly Arg Ala Ile Thr Ala Ser Glu Ala Arg
        355                 360                 365

Arg Ile Arg Pro Arg Ala Val Arg Ala Phe Val Asp Arg Leu Pro Arg
    370                 375                 380

Val Thr Arg Arg Arg Arg Arg Pro Pro Ser Pro Ala Pro Pro Pro Glu
385                 390                 395                 400

Glu Ile Glu Glu Ala Ala Met Glu Val Glu Glu Pro Glu Glu Glu Glu
                405                 410                 415

Glu Glu Leu Leu Asp Glu Val Ile Arg Thr Ala Leu Glu Ala Ile Gly
            420                 425                 430

Ala Leu Gln Asp Glu Leu Ser Gly Ala Ala Arg Arg His Glu Leu Phe
        435                 440                 445

Arg Phe Ala Asn Asp Phe Tyr Arg Met Leu Leu Thr Ala Arg Asp Ala
    450                 455                 460

Gly Leu Met Gly Glu Ser Phe Leu Arg Lys Trp Val Leu Tyr Phe Phe

465                 470                 475                 480

Leu Ala Glu His Ile Ala Ser Thr Leu Tyr Tyr Leu Tyr Ser His Phe
                485                 490                 495

Ile Ala Asn Arg Glu Phe Arg Arg Tyr Val Asp Val Leu Thr Leu Gln
            500                 505                 510

Val Leu Val Val Gly Trp Asp Val Asn Ala Gln Gln Val Phe Lys Arg
        515                 520                 525

Ile Trp Ser Glu Gln Ser Asn Pro Ala Thr Ile Phe Glu Thr Leu Trp
    530                 535                 540

Glu Arg Ile Leu Arg Asp Phe Leu Met Met Val Glu Arg Thr Gly Gln
545                 550                 555                 560

Phe Glu Gly Met Asp Asp Ala Asp Gln Gln Leu Phe Leu Ser Asp Ile
                565                 570                 575

Gln Tyr Arg Asp Arg Ser Gly Asp Ile Glu Glu Val Leu Lys Gln Leu
            580                 585                 590

Asn Leu Ser Glu Glu Leu Ile Asp Ser Ile Asp Ile Ser Phe Arg Ile
        595                 600                 605

Lys Phe Lys Gly Ile Val Ala Ile Ala Thr Asn Glu Glu Ile Lys Ala
    610                 615                 620

Asn Leu Arg Arg Val Leu Arg His Arg Arg Glu Asp Ile Glu Ala Ala
625                 630                 635                 640

Ala Arg Arg Gly Gln Pro Leu
                645

<210> 52
<211> 1977
<212> DNA
<213> adenoviridae

<220>
<223> Description of Combined DNA/RNA Molecule: Human
      Ad5/Avian Ad Celo Phelps HpTP

<220>
<221> CDS
<222> (1)..(1977)

<400> 52
atg gcc ttg agc gtc aac gat tgc gcg cgc ctg acc ggc cag agc gtc      48
Met Ala Leu Ser Val Asn Asp Cys Ala Arg Leu Thr Gly Gln Ser Val
  1               5                  10                  15

ccg acc atg gag cac ttt ttg ccg ctg cgc aac atc tgg aac cgc gtc      96
Pro Thr Met Glu His Phe Leu Pro Leu Arg Asn Ile Trp Asn Arg Val
             20                  25                  30

cgc gac ttt ccg cgc gcc tcc acc acc gcc gcc ggc atc acc tgg atg     144
Arg Asp Phe Pro Arg Ala Ser Thr Thr Ala Ala Gly Ile Thr Trp Met
         35                  40                  45

tcc agg tac atc tac gga tat cat cgc ctt atg ttg gaa gat ctc gcc     192
Ser Arg Tyr Ile Tyr Gly Tyr His Arg Leu Met Leu Glu Asp Leu Ala
     50                  55                  60

ccc gga gcc ccg gcc acc cta cgc tgg ccc ctc tac cgc cag ccg ccg 240
Pro Gly Ala Pro Ala Thr Leu Arg Trp Pro Leu Tyr Arg Gln Pro Pro
65                  70                  75                  80

ccg cac ttt ttg gtg gga tac cag tac ctg gtg cgg act tgc aac gac 288
Pro His Phe Leu Val Gly Tyr Gln Tyr Leu Val Arg Thr Cys Asn Asp
                85                  90                  95

tac gta ttt gac tcg agg gct tac tcg cgt ctc agg tac acc gag ctc 336
Tyr Val Phe Asp Ser Arg Ala Tyr Ser Arg Leu Arg Tyr Thr Glu Leu
            100                 105                 110

tcg cag ccg ggt cac cag acc gtt aac tgg tcc gtt atg gcc aac tgc 384
Ser Gln Pro Gly His Gln Thr Val Asn Trp Ser Val Met Ala Asn Cys
        115                 120                 125

act tac acc atc aac acg ggc gca tac cac cgc ttt gtg gac atg gat 432
Thr Tyr Thr Ile Asn Thr Gly Ala Tyr His Arg Phe Val Asp Met Asp
    130                 135                 140

gac ttc cag tct acc ctc acg cag gtg cag cag gcc ata tta gcc gag 480
Asp Phe Gln Ser Thr Leu Thr Gln Val Gln Gln Ala Ile Leu Ala Glu
145                 150                 155                 160

cgc gtt gtc gcc gac cta gcc ctg ctt cag ccg atg agg ggc ttc ggg 528
Arg Val Val Ala Asp Leu Ala Leu Leu Gln Pro Met Arg Gly Phe Gly
                165                 170                 175

gtc aca cgc atg gga gga aga ggg cgc cac cta cgg cca aac tcc gcc 576
Val Thr Arg Met Gly Gly Arg Gly Arg His Leu Arg Pro Asn Ser Ala
            180                 185                 190

gcc gcc gca gcg ata gat gca aga gat gca gga caa gag gaa gga gaa 624
Ala Ala Ala Ala Ile Asp Ala Arg Asp Ala Gly Gln Glu Glu Gly Glu
        195                 200                 205

gaa gaa gtg ccg gta gaa agg ctc atg caa gac tac tac aaa gac ctg 672
Glu Glu Val Pro Val Glu Arg Leu Met Gln Asp Tyr Tyr Lys Asp Leu
    210                 215                 220

cgc cga tgt caa aac gaa gcc tgg ggc atg gcc gac cgc ctg cgc att 720
Arg Arg Cys Gln Asn Glu Ala Trp Gly Met Ala Asp Arg Leu Arg Ile
225                 230                 235                 240

cag cag gcc gga ccc aag gac atg gtg ctt ctg tcg acc atc cgc cgt 768
Gln Gln Ala Gly Pro Lys Asp Met Val Leu Leu Ser Thr Ile Arg Arg
                245                 250                 255

ctc aag acc gcc tac ttt aat tac atc atc agc agc acc tcc gcc aga 816
Leu Lys Thr Ala Tyr Phe Asn Tyr Ile Ile Ser Ser Thr Ser Ala Arg
            260                 265                 270

aac aac ccc gac cgc cgc ccg ctg ccg ccc gcc acg gtg ctc agc cta 864
Asn Asn Pro Asp Arg Arg Pro Leu Pro Pro Ala Thr Val Leu Ser Leu
        275                 280                 285

cct tgc gac tgt gac tgg tta gac gcc ttt ctc gag agg ttt tcc gat 912
Pro Cys Asp Cys Asp Trp Leu Asp Ala Phe Leu Glu Arg Phe Ser Asp
    290                 295                 300

ccg gtc gat gcg gac tcg ctc agg tcc ctc ggc ggc gga gta cct aca 960
Pro Val Asp Ala Asp Ser Leu Arg Ser Leu Gly Gly Gly Val Pro Thr
305                 310                 315                 320

caa caa ttg ttg aga tgc atc gtt agc gcc gta tcc ctg ccg cat ggc

1008
Gln Gln Leu Leu Arg Cys Ile Val Ser Ala Val Ser Leu Pro His Gly
                325                 330                 335

agc ccc ccg cca acc cat aac cgg gac atg acg ggc ggc gtc ttc caa
1056
Ser Pro Pro Pro Thr His Asn Arg Asp Met Thr Gly Gly Val Phe Gln
            340                 345                 350

ctc tgc agc cgg cgg gta acc gac cgc acc ggc ctg cgc cct aga gac
1104
Leu Cys Ser Arg Arg Val Thr Asp Arg Thr Gly Leu Arg Pro Arg Asp
        355                 360                 365

cgc cac ggc aga gcc atc acc gcg tcc gaa gcg cgc cgc att agg ccc
1152
Arg His Gly Arg Ala Ile Thr Ala Ser Glu Ala Arg Arg Ile Arg Pro
    370                 375                 380

cgt gcc gtg cgg gcc ttc gta gac cgc ctg ccc cgc gtc acg cgg cgg
1200
Arg Ala Val Arg Ala Phe Val Asp Arg Leu Pro Arg Val Thr Arg Arg
385                 390                 395                 400

cga cgg aga ccc ccc tcc ccc gcg ccc cct ccc gaa gaa ata gaa gaa
1248
Arg Arg Arg Pro Pro Ser Pro Ala Pro Pro Pro Glu Glu Ile Glu Glu
                405                 410                 415

gcc gcc atg gaa gta gaa gaa cca gaa gag gag gaa gaa gag ctg tta
1296
Ala Ala Met Glu Val Glu Glu Pro Glu Glu Glu Glu Glu Glu Leu Leu
            420                 425                 430

gac gag gtg att cgc aca gcg ctc gaa gcc atc ggg gca ctg caa gac
1344
Asp Glu Val Ile Arg Thr Ala Leu Glu Ala Ile Gly Ala Leu Gln Asp
        435                 440                 445

gag ctc agc ggg gcc gcc cgg aga cac gaa ctc ttc agg ttt gcc aac
1392
Glu Leu Ser Gly Ala Ala Arg Arg His Glu Leu Phe Arg Phe Ala Asn
    450                 455                 460

gac ttc tac cgc atg ctc ctg acc gcg cgc gac gcg gga ctc atg gga
1440
Asp Phe Tyr Arg Met Leu Leu Thr Ala Arg Asp Ala Gly Leu Met Gly
465                 470                 475                 480

gag tcg ttc ctg cgc aag tgg gtg ctg tac ttc ttc tta gcc gaa cat
1488
Glu Ser Phe Leu Arg Lys Trp Val Leu Tyr Phe Phe Leu Ala Glu His
                485                 490                 495

atc gcc tct aca ctc tac tac ctg tac agc cac ttc atc gcc aac cgc
1536
Ile Ala Ser Thr Leu Tyr Tyr Leu Tyr Ser His Phe Ile Ala Asn Arg
            500                 505                 510

gag ttc cgc cgg tac gtc gac gtc ctg acc tta caa gta ctc gtc gtg
1584
Glu Phe Arg Arg Tyr Val Asp Val Leu Thr Leu Gln Val Leu Val Val
        515                 520                 525

gga tgg gac gtc aac gcg cag cag gtt ttt aaa cgc ata tgg agc gag
1632

Gly Trp Asp Val Asn Ala Gln Gln Val Phe Lys Arg Ile Trp Ser Glu
    530                 535                 540

caa tcc aac ccc gcc acc ata ttc gaa acc ctg tgg gaa cgt ata tta
1680
Gln Ser Asn Pro Ala Thr Ile Phe Glu Thr Leu Trp Glu Arg Ile Leu
545                 550                 555                 560

cgc gat ttt ctt atg atg gtc gaa cgg acg ggt caa ttc gaa ggc atg
1728
Arg Asp Phe Leu Met Met Val Glu Arg Thr Gly Gln Phe Glu Gly Met
                565                 570                 575

gac gat gcg gac caa caa ctg ttt ctc tct gat att caa tac aga gac
1776
Asp Asp Ala Asp Gln Gln Leu Phe Leu Ser Asp Ile Gln Tyr Arg Asp
            580                 585                 590

cgc tcc ggt gac atc gaa gaa gtg ctg aag cag ctc aac ctc agc gaa
1824
Arg Ser Gly Asp Ile Glu Glu Val Leu Lys Gln Leu Asn Leu Ser Glu
        595                 600                 605

gag ctg atc gat agc atc gac atc agt ttc cgc atc aaa ttt aaa ggc
1872
Glu Leu Ile Asp Ser Ile Asp Ile Ser Phe Arg Ile Lys Phe Lys Gly
    610                 615                 620

atc gta gcc atc gct acc aac gag gag atc aaa gcc aac ctc aga cgc
1920
Ile Val Ala Ile Ala Thr Asn Glu Glu Ile Lys Ala Asn Leu Arg Arg
625                 630                 635                 640

gtg ctc cgc cac cgc cgc gaa gac atc gaa gcg gcg gcg cga cga ggt
1968
Val Leu Arg His Arg Arg Glu Asp Ile Glu Ala Ala Ala Arg Arg Gly
                645                 650                 655

cag cct ctg
1977
Gln Pro Leu


<210> 53
<211> 659
<212> PRT
<213> adenoviridae
<223> Description of Combined DNA/RNA Molecule: Human
      Ad5/Avian Ad Celo Phelps HpTP

<400> 53
Met Ala Leu Ser Val Asn Asp Cys Ala Arg Leu Thr Gly Gln Ser Val
  1               5                  10                  15

Pro Thr Met Glu His Phe Leu Pro Leu Arg Asn Ile Trp Asn Arg Val
             20                  25                  30

Arg Asp Phe Pro Arg Ala Ser Thr Thr Ala Ala Gly Ile Thr Trp Met
         35                  40                  45

Ser Arg Tyr Ile Tyr Gly Tyr His Arg Leu Met Leu Glu Asp Leu Ala
     50                  55                  60

Pro Gly Ala Pro Ala Thr Leu Arg Trp Pro Leu Tyr Arg Gln Pro Pro
 65                  70                  75                  80

Pro His Phe Leu Val Gly Tyr Gln Tyr Leu Val Arg Thr Cys Asn Asp
                85                  90                  95

Tyr Val Phe Asp Ser Arg Ala Tyr Ser Arg Leu Arg Tyr Thr Glu Leu
            100                 105                 110

Ser Gln Pro Gly His Gln Thr Val Asn Trp Ser Val Met Ala Asn Cys
        115                 120                 125

Thr Tyr Thr Ile Asn Thr Gly Ala Tyr His Arg Phe Val Asp Met Asp
    130                 135                 140

Asp Phe Gln Ser Thr Leu Thr Gln Val Gln Gln Ala Ile Leu Ala Glu
145                 150                 155                 160

Arg Val Val Ala Asp Leu Ala Leu Leu Gln Pro Met Arg Gly Phe Gly
                165                 170                 175

Val Thr Arg Met Gly Gly Arg Gly Arg His Leu Arg Pro Asn Ser Ala
            180                 185                 190

Ala Ala Ala Ala Ile Asp Ala Arg Asp Ala Gly Gln Glu Glu Gly Glu
        195                 200                 205

Glu Glu Val Pro Val Glu Arg Leu Met Gln Asp Tyr Tyr Lys Asp Leu
    210                 215                 220

Arg Arg Cys Gln Asn Glu Ala Trp Gly Met Ala Asp Arg Leu Arg Ile
225                 230                 235                 240

Gln Gln Ala Gly Pro Lys Asp Met Val Leu Leu Ser Thr Ile Arg Arg
                245                 250                 255

Leu Lys Thr Ala Tyr Phe Asn Tyr Ile Ile Ser Ser Thr Ser Ala Arg
            260                 265                 270

Asn Asn Pro Asp Arg Arg Pro Leu Pro Pro Ala Thr Val Leu Ser Leu
        275                 280                 285

Pro Cys Asp Cys Asp Trp Leu Asp Ala Phe Leu Glu Arg Phe Ser Asp
    290                 295                 300

Pro Val Asp Ala Asp Ser Leu Arg Ser Leu Gly Gly Gly Val Pro Thr
305                 310                 315                 320

Gln Gln Leu Leu Arg Cys Ile Val Ser Ala Val Ser Leu Pro His Gly
                325                 330                 335

Ser Pro Pro Pro Thr His Asn Arg Asp Met Thr Gly Gly Val Phe Gln
            340                 345                 350

Leu Cys Ser Arg Arg Val Thr Asp Arg Thr Gly Leu Arg Pro Arg Asp
        355                 360                 365

Arg His Gly Arg Ala Ile Thr Ala Ser Glu Ala Arg Arg Ile Arg Pro
    370                 375                 380

Arg Ala Val Arg Ala Phe Val Asp Arg Leu Pro Arg Val Thr Arg Arg
385                 390                 395                 400

Arg Arg Arg Pro Pro Ser Pro Ala Pro Pro Pro Glu Glu Ile Glu Glu
                405                 410                 415

Ala Ala Met Glu Val Glu Glu Pro Glu Glu Glu Glu Glu Leu Leu
            420                 425                 430

Asp Glu Val Ile Arg Thr Ala Leu Glu Ala Ile Gly Ala Leu Gln Asp
            435                 440                 445

Glu Leu Ser Gly Ala Ala Arg Arg His Glu Leu Phe Arg Phe Ala Asn
        450                 455                 460

Asp Phe Tyr Arg Met Leu Leu Thr Ala Arg Asp Ala Gly Leu Met Gly
465 470 475 480

Glu Ser Phe Leu Arg Lys Trp Val Leu Tyr Phe Phe Leu Ala Glu His
485 490 495

Ile Ala Ser Thr Leu Tyr Tyr Leu Tyr Ser His Phe Ile Ala Asn Arg
                500                 505                 510

Glu Phe Arg Arg Tyr Val Asp Val Leu Thr Leu Gln Val Leu Val Val
            515                 520                 525

Gly Trp Asp Val Asn Ala Gln Gln Val Phe Lys Arg Ile Trp Ser Glu
        530                 535                 540

Gln Ser Asn Pro Ala Thr Ile Phe Glu Thr Leu Trp Glu Arg Ile Leu
545 550 555 560

Arg Asp Phe Leu Met Met Val Glu Arg Thr Gly Gln Phe Glu Gly Met
565 570 575

Asp Asp Ala Asp Gln Gln Leu Phe Leu Ser Asp Ile Gln Tyr Arg Asp
                580                 585                 590

Arg Ser Gly Asp Ile Glu Glu Val Leu Lys Gln Leu Asn Leu Ser Glu
            595                 600                 605

Glu Leu Ile Asp Ser Ile Asp Ile Ser Phe Arg Ile Lys Phe Lys Gly
        610                 615                 620

Ile Val Ala Ile Ala Thr Asn Glu Glu Ile Lys Ala Asn Leu Arg Arg
625 630 635 640

Val Leu Arg His Arg Arg Glu Asp Ile Glu Ala Ala Ala Arg Arg Gly
645 650 655

Gln Pro Leu

<210> 54
<211> 1782
<212> DNA
<213> adenoviridae

<220>
<223> Description of Combined DNA/RNA Molecule: Other
Celo phelps pTP sequence containging Ad5 part
upstream of ORF

<220>
<221> CDS
<222> (1)..(1779)

<400> 54
   atg gcc ttg agc gtc aac gat tgc gcg cgc ctg acc ggc cag agc gtc 48
   Met Ala Leu Ser Val Asn Asp Cys Ala Arg Leu Thr Gly Gln Ser Val
   1 5 10 15
   ccg acc atg caa ctc cga gac ctc gcg ccg cga tcg ccg aac gtc gcc 96
   Pro Thr Met Gln Leu Arg Asp Leu Ala Pro Arg Ser Pro Asn Val Ala
   20 25 30
   gcg ccg ccc tac aac gga ttg ccg ccg ccg cac ctt ctc ctc ggg tac   144
   Ala Pro Pro Tyr Asn Gly Leu Pro Pro Pro His Leu Leu Leu Gly Tyr
           35                  40                  45
   caa gct atg cac cgc gcg ctc aac gat tac ctt ttc gac aac cgc gtt 192
   Gln Ala Met His Arg Ala Leu Asn Asp Tyr Leu Phe Asp Asn Arg Val
   50 55 60
   ttt atg cag ata ggt tac gat agc cca ccc caa aga ccc aga cgc ctc 240
   Phe Met Gln Ile Gly Tyr Asp Ser Pro Pro Gln Arg Pro Arg Arg Leu
   65 70 75 80
   ttt tgg acc tgt ctg acc gac tgc tcc tac gcc gtc aat gta ggg cag 288
   Phe Trp Thr Cys Leu Thr Asp Cys Ser Tyr Ala Val Asn Val Gly Gln
   85 90 95
   tac atg cga ttt ctc gat ctc gac aac ttt cac ggt acg ttc acg cag 336
   Tyr Met Arg Phe Leu Asp Leu Asp Asn Phe His Gly Thr Phe Thr Gln
   100 105 110
   atg cac aac gcc gta ctc atg gac cgc gtg gcc gcg gac atg ggc cgg 384
   Met His Asn Ala Val Leu Met Asp Arg Val Ala Ala Asp Met Gly Arg
   115 120 125
   gcg cat ctg cga ggt agg gga atc gac gta ggc cgt cac gga caa gtg 432
   Ala His Leu Arg Gly Arg Gly Ile Asp Val Gly Arg His Gly Gln Val
   130   135 140
   ttg ccg cag ctc gac gcc gaa cac cac agc cta ctg tcg ggc aac gga 480
   Leu Pro Gln Leu Asp Ala Glu His His Ser Leu Leu Ser Gly Asn Gly
   145 150 155 160
   gcg ggt ggc ttg caa gaa ggc gtc ctc atg cga acg gcc tct gcc gcc 528
   Ala Gly Gly Leu Gln Glu Gly Val Leu Met Arg Thr Ala Ser Ala Ala
   165 170 175
   gac gcc gaa ctg ctc gcc gcc atc cgc caa cta aga gtc gcc ctc tgc 576
   Asp Ala Glu Leu Leu Ala Ala Ile Arg Gln Leu Arg Val Ala Leu Cys
   180 185 190
   cac tat cta ttc tgc tac gca tat gat cta ttt caa acg gaa gaa aga 624
   His Tyr Leu Phe Cys Tyr Ala Tyr Asp Leu Phe Gln Thr Glu Glu Arg
   195 200 205
   tat cgg ttc tta cct gga tcc gat gtg ttc ctt gaa cca aac tgg ctc 672
   Tyr Arg Phe Leu Pro Gly Ser Asp Val Phe Leu Glu Pro Asn Trp Leu
   210 215 220
   tcc tac ttc gcg gaa gcc ttc gcg gag cta gac acc cag caa ctg gtg 720
   Ser Tyr Phe Ala Glu Ala Phe Ala Glu Leu Asp Thr Gln Gln Leu Val
   225 230 235 240
   cgg gat gcc gag cgc aag ttt cga gga aga cgg gac gta gag gaa cct 768
   Arg Asp Ala Glu Arg Lys Phe Arg Gly Arg Arg Asp Val Glu Glu Pro
   245 250 255
   acg gaa aca atg gcg aga tgt ttc atg agc act cta gcg agc gac gcc 816
   Thr Glu Thr Met Ala Arg Cys Phe Met Ser Thr Leu Ala Ser Asp Ala
   260 265 270
   gtt tcc tta gca gga acg ggt ctg tca gga ggc gcc atc acc ctc tgc 864
   Val Ser Leu Ala Gly Thr Gly Leu Ser Gly Gly Ala Ile Thr Leu Cys
   275                 280                 285
   agc cgg cgg gta acc gac cgc acc ggc ctg cgc cct aga gac cgc cac     912
   Ser Arg Arg Val Thr Asp Arg Thr Gly Leu Arg Pro Arg Asp Arg His
       290                 295                 300
   ggc aga gcc atc acc gcg tcc gaa gcg cgc cgc att agg ccc cgt gcc     960
   Gly Arg Ala Ile Thr Ala Ser Glu Ala Arg Arg Ile Arg Pro Arg Ala
   305                 310                 315                 320
   gtg cgg gcc ttc gta gac cgc ctg ccc cgc gtc acg cgg cgg cga cgg
   1008
   Val Arg Ala Phe Val Asp Arg Leu Pro Arg Val Thr Arg Arg Arg Arg
                   325                 330                 335
   aga ccc ccc tcc ccc gcg ccc cct ccc gaa gaa ata gaa gaa gcc gcc
   1056
   Arg Pro Pro Ser Pro Ala Pro Pro Pro Glu Glu Ile Glu Glu Ala Ala
               340                 345                 350
   atg gaa gta gaa gaa cca gaa gag gag gaa gaa gag ctg tta gac gag
   1104
   Met Glu Val Glu Glu Pro Glu Glu Glu Glu Glu Glu Leu Leu Asp Glu
           355                 360                 365
   gtg att cgc aca gcg ctc gaa gcc atc ggg gca ctg caa gac gag ctc
   1152
   Val Ile Arg Thr Ala Leu Glu Ala Ile Gly Ala Leu Gln Asp Glu Leu
       370                 375                 380
   agc ggg gcc gcc cgg aga cac gaa ctc ttc agg ttt gcc aac gac ttc
   1200
   Ser Gly Ala Ala Arg Arg His Glu Leu Phe Arg Phe Ala Asn Asp Phe
   385                 390                 395                 400
   tac cgc atg ctc ctg acc gcg cgc gac gcg gga ctc atg gga gag tcg
   1248
   Tyr Arg Met Leu Leu Thr Ala Arg Asp Ala Gly Leu Met Gly Glu Ser
                   405                 410                 415
   ttc ctg cgc aag tgg gtg ctg tac ttc ttc tta gcc gaa cat atc gcc
   1296
   Phe Leu Arg Lys Trp Val Leu Tyr Phe Phe Leu Ala Glu His Ile Ala
               420                 425                 430
   tct aca ctc tac tac ctg tac agc cac ttc atc gcc aac cgc gag ttc
   1344
   Ser Thr Leu Tyr Tyr Leu Tyr Ser His Phe Ile Ala Asn Arg Glu Phe
           435                 440                 445
   cgc cgg tac gtc gac gtc ctg acc tta caa gta ctc gtc gtg gga tgg
   1392
   Arg Arg Tyr Val Asp Val Leu Thr Leu Gln Val Leu Val Val Gly Trp
       450                 455                 460
   gac gtc aac gcg cag cag gtt ttt aaa cgc ata tgg agc gag caa tcc
   1440
   Asp Val Asn Ala Gln Gln Val Phe Lys Arg Ile Trp Ser Glu Gln Ser
   465                 470                 475                 480
   aac ccc gcc acc ata ttc gaa acc ctg tgg gaa cgt ata tta cgc gat
   1488
   Asn Pro Ala Thr Ile Phe Glu Thr Leu Trp Glu Arg Ile Leu Arg Asp
                   485                 490                 495
   ttt ctt atg atg gtc gaa cgg acg ggt caa ttc gaa ggc atg gac gat
   1536
   Phe Leu Met Met Val Glu Arg Thr Gly Gln Phe Glu Gly Met Asp Asp
               500                 505                 510
   gcg gac caa caa ctg ttt ctc tct gat att caa tac aga gac cgc tcc
   1584
   Ala Asp Gln Gln Leu Phe Leu Ser Asp Ile Gln Tyr Arg Asp Arg Ser
           515                 520                 525
   ggt gac atc gaa gaa gtg ctg aag cag ctc aac ctc agc gaa gag ctg
   1632
   Gly Asp Ile Glu Glu Val Leu Lys Gln Leu Asn Leu Ser Glu Glu Leu
       530                 535                 540
   atc gat agc atc gac atc agt ttc cgc atc aaa ttt aaa ggc atc gta
   1680
   Ile Asp Ser Ile Asp Ile Ser Phe Arg Ile Lys Phe Lys Gly Ile Val
   545                 550                 555                 560
   gcc atc gct acc aac gag gag atc aaa gcc aac ctc aga cgc gtg ctc
   1728
   Ala Ile Ala Thr Asn Glu Glu Ile Lys Ala Asn Leu Arg Arg Val Leu
                   565                 570                 575
   cgc cac cgc cgc gaa gac atc gaa gcg gcg gcg cga cga ggt cag cct
   1776
   Arg His Arg Arg Glu Asp Ile Glu Ala Ala Ala Arg Arg Gly Gln Pro
               580                 585                 590
   ctg taa
   1782
   Leu


<210> 55
<211> 593
<212> PRT
<213> adenoviridae
<223> Description of Combined DNA/RNA Molecule: Other
      Celo phelps pTP sequence containging Ad5 part
      upstream of ORF

<400> 55
   Met Ala Leu Ser Val Asn Asp Cys Ala Arg Leu Thr Gly Gln Ser Val
     1               5                  10                  15
   Pro Thr Met Gln Leu Arg Asp Leu Ala Pro Arg Ser Pro Asn Val Ala
                20                  25                  30
   Ala Pro Pro Tyr Asn Gly Leu Pro Pro Pro His Leu Leu Leu Gly Tyr
            35                  40                  45
   Gln Ala Met His Arg Ala Leu Asn Asp Tyr Leu Phe Asp Asn Arg Val
        50                  55                  60
   Phe Met Gln Ile Gly Tyr Asp Ser Pro Pro Gln Arg Pro Arg Arg Leu
    65                  70                  75                  80
   Phe Trp Thr Cys Leu Thr Asp Cys Ser Tyr Ala Val Asn Val Gly Gln
                    85                  90                  95
   Tyr Met Arg Phe Leu Asp Leu Asp Asn Phe His Gly Thr Phe Thr Gln
               100                 105                 110
   Met His Asn Ala Val Leu Met Asp Arg Val Ala Ala Asp Met Gly Arg
           115                 120                 125
   Ala His Leu Arg Gly Arg Gly Ile Asp Val Gly Arg His Gly Gln Val
       130                 135                 140
   Leu Pro Gln Leu Asp Ala Glu His His Ser Leu Leu Ser Gly Asn Gly
   145                 150                 155                 160
   Ala Gly Gly Leu Gln Glu Gly Val Leu Met Arg Thr Ala Ser Ala Ala
                   165                 170                 175
   Asp Ala Glu Leu Leu Ala Ala Ile Arg Gln Leu Arg Val Ala Leu Cys
               180                 185                 190
   His Tyr Leu Phe Cys Tyr Ala Tyr Asp Leu Phe Gln Thr Glu Glu Arg
           195                 200                 205
   Tyr Arg Phe Leu Pro Gly Ser Asp Val Phe Leu Glu Pro Asn Trp Leu
       210                 215                 220
   Ser Tyr Phe Ala Glu Ala Phe Ala Glu Leu Asp Thr Gln Gln Leu Val
   225                 230                 235                 240
   Arg Asp Ala Glu Arg Lys Phe Arg Gly Arg Arg Asp Val Glu Glu Pro
                   245                 250                 255
   Thr Glu Thr Met Ala Arg Cys Phe Met Ser Thr Leu Ala Ser Asp Ala
               260                 265                 270
   Val Ser Leu Ala Gly Thr Gly Leu Ser Gly Gly Ala Ile Thr Leu Cys
           275                 280                 285
   Ser Arg Arg Val Thr Asp Arg Thr Gly Leu Arg Pro Arg Asp Arg His
       290                 295                 300
   Gly Arg Ala Ile Thr Ala Ser Glu Ala Arg Arg Ile Arg Pro Arg Ala
   305                 310                 315                 320
   Val Arg Ala Phe Val Asp Arg Leu Pro Arg Val Thr Arg Arg Arg Arg
                   325                 330                 335
   Arg Pro Pro Ser Pro Ala Pro Pro Pro Glu Glu Ile Glu Glu Ala Ala
               340                 345                 350
   Met Glu Val Glu Glu Pro Glu Glu Glu Glu Glu Glu Leu Leu Asp Glu
           355                 360                 365
   Val Ile Arg Thr Ala Leu Glu Ala Ile Gly Ala Leu Gln Asp Glu Leu
       370                 375                 380
   Ser Gly Ala Ala Arg Arg His Glu Leu Phe Arg Phe Ala Asn Asp Phe
   385                 390                 395                 400
   Tyr Arg Met Leu Leu Thr Ala Arg Asp Ala Gly Leu Met Gly Glu Ser
                   405                 410                 415
   Phe Leu Arg Lys Trp Val Leu Tyr Phe Phe Leu Ala Glu His Ile Ala
               420                 425                 430
   Ser Thr Leu Tyr Tyr Leu Tyr Ser His Phe Ile Ala Asn Arg Glu Phe
           435                 440                 445
   Arg Arg Tyr Val Asp Val Leu Thr Leu Gln Val Leu Val Val Gly Trp
       450                 455                 460
   Asp Val Asn Ala Gln Gln Val Phe Lys Arg Ile Trp Ser Glu Gln Ser
   465                 470                 475                 480
   Asn Pro Ala Thr Ile Phe Glu Thr Leu Trp Glu Arg Ile Leu Arg Asp
   485 490 495
   Phe Leu Met Met Val Glu Arg Thr Gly Gln Phe Glu Gly Met Asp Asp
   500 505 510
   Ala Asp Gln Gln Leu Phe Leu Ser Asp Ile Gln Tyr Arg Asp Arg Ser
   515 520 525
   Gly Asp Ile Glu Glu Val Leu Lys Gln Leu Asn Leu Ser Glu Glu Leu
   530 535 540
   Ile Asp Ser Ile Asp Ile Ser Phe Arg Ile Lys Phe Lys Gly Ile Val
   545 550 555 560
   Ala Ile Ala Thr Asn Glu Glu Ile Lys Ala Asn Leu Arg Arg Val Leu
   565 570 575
   Arg His Arg Arg Glu Asp Ile Glu Ala Ala Ala Arg Arg Gly Gln Pro
   580 585 590
   Leu